# EUROPÄISCHE PATENTSCHRIFT

(11) **EP 2 529 632 B1**
(45) Veröffentlichungstag und Bekanntmachung des Hinweises auf die Patenterteilung: **28.08.2013**
(21) Anmeldenummer: 11168310.8
(22) Anmeldetag: 31.05.2011
(51) Int. Cl.: A23L 1/22

(54) **Zimtsäureamide als würzige Geschmacksstoffe**
Cinnamic acid amides as spicy flavour agents
Acide aminé cinnamique comme arômes épicés

(43) Veröffentlichungstag der Anmeldung: 05.12.2012
(73) Patentinhaber: Symrise AG, 37603 Holzminden (DE)
(72) Erfinder: Ley, Jakob, Dr., 37603 Holzminden (DE); Reichelt, Katharina, Dr., 37603 Holzminden (DE); Paetz, Susanne, 37671 Höxter (DE); Backes, Michael, Dr., 37603 Holzminden (DE); Obst, Katja, 37603 Holzminden (DE)
(74) Vertreter: Eisenführ, Speiser & Partner

(56) Entgegenhaltungen:
- EP-A1- 2 064 959
- US-A1- 2004 202 619
- ADESINA S K ET AL: "Amides from Zanthoxylum rubescens", PHYTOCHEMISTRY, PERGAMON PRESS, GB, Bd. 28, Nr. 3, 1. Januar 1989 (1989-01-01) , Seiten 839-842, XP026616404, ISSN: 0031-9422, DOI: 10.1016/0031-9422(89)80125-6 [gefunden am 1989-01-01]
- BASSARD J E ET AL: "Phenolamides: Bridging polyamines to the phenolic metabolism", PHYTOCHEMISTRY, PERGAMON PRESS, GB, Bd. 71, Nr. 16, 1. November 2010 (2010-11-01), Seiten 1808-1824, XP027406649, ISSN: 0031-9422 [gefunden am 2010-08-26]

## Beschreibung

Die vorliegende Erfindung betrifft primär die Verwendung von Verbindungen der unten gezeigten Formel (I) bzw. Mischungen umfassend oder bestehend aus zwei oder mehr Verbindungen der Formel (I) als Geschmacksstoff bzw. Geschmacksstoffmischung zum Vermitteln, Modifizieren und/oder Verstärken einer, zwei oder sämtlicher der Geschmackseindrücke Umami, Kokumi und salzig, 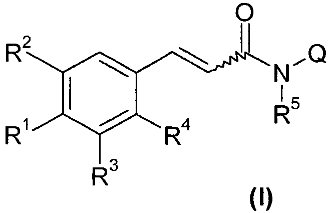 wobei für die bzw. jede Verbindung der Formel (I) hinsichtlich der Bedeutung der Reste bzw. Gruppen R¹ bis R⁵ und Q das in der folgenden Beschreibung sowie in den Ansprüchen Gesagte gilt.

Zudem betrifft die vorliegende Erfindung Geschmacksstoffmischungen umfassend oder bestehend aus einer oder mehreren erfindungsgemäß zu verwendenden Verbindung(en) der Formel (I) sowie einem, zwei, drei oder mehreren weiteren Geschmacksstoffen zum Vermitteln, Modifizieren und/oder Verstärken einer, zwei oder sämtlicher der Geschmackseindrücke Umami, Kokumi und salzig, wobei der bzw. die weiteren Geschmacksstoffe keine Verbindungen der Formel (I) sind.

Die vorliegende Erfindung betrifft zudem einen pflanzlichen Extrakt umfassend oder bestehend aus einer oder mehreren erfindungsgemäß zu verwendenden Verbindung(en) der Formel (I) sowie einem oder mehreren weiteren Bestandteilen, wobei die Gesamtmenge an Verbindungen der Formel (I) bezogen auf das Gesamtgewicht des pflanzlichen Extrakts im Bereich von 1.000 bis 500.000 ppm, vorzugsweise im Bereich von 10.000 bis 100.000 ppm liegt. Die vorliegende Erfindung betrifft auch die Verwendung eines solchen Extrakts zum Vermitteln, Modifizieren und/oder Verstärken einer, zwei oder sämtlicher der Geschmackseindrücke Umami, Kokumi und salzig.

Des Weiteren betrifft die vorliegende Erfindung der Ernährung oder dem Genuss dienende (gebrauchs- oder verzehrfertige) Zubereitungen, die aus einem oder mehreren zum Verzehr geeigneten Bestandteilen, welche keine Verbindungen der Formel (I) sind, sowie einer oder mehreren Verbindung(en) der Formel (I) bzw. einer erfindungsgemäßen Geschmacksstoffmischung oder einem erfindungsgemäßen pflanzlichen Extrakt umfassen oder daraus bestehen.

Des Weiteren betrifft die vorliegende Erfindung auch Halbfertigwaren zur Herstellung einer der Ernährung oder dem Genuss dienenden Zubereitung, vorzugsweise einer erfindungsgemäßen Zubereitung.

Weitere Aspekte der vorliegenden Erfindung ergeben sich aus der folgenden Beschreibung, den Beispielen, den Figuren sowie den beigefügten Patentansprüchen.

Es besteht ein ständiger Bedarf, neue Geschmacksstoffe, d.h. geschmacksaktive Verbindungen bzw. Verbindungen, die einen Geschmackseindruck vermitteln, modifizieren und/oder verstärken können, aufzufinden. Insbesondere besteht Bedarf an solchen Verbindungen, die den Geschmackseindruck Umami und/oder Kokumi vermitteln (erzeugen), modifizieren und/oder verstärken können. Im Zuge des verstärkten Gesundheitsbewusstseins der Konsumenten werden zudem Verbindungen gesucht, die einen salzigen Geschmackseindruck vermitteln, modifizieren und/oder verstärken können. Insgesamt besteht demnach ein besonderer Bedarf an würzigen Geschmacksstoffen, die sämtliche der Geschmackseindrücke Umami, Kokumi und salzig vermitteln, modifizieren und/oder verstärken können. Besonders bevorzugt sind im allgemeinen Geschmacksstoffe, die in natürlichen, frischen, getrockneten oder gegebenenfalls mit für Lebensmittel üblichen Zubereitungsarten (z.B. Evaporation oder Pervaporation, Extraktion, Dämpfen, Erhitzen, Rösten, Kochen, Braten, Kühlen, Mahlen, Enzym-Behandlung, Fermentieren) behandelten Quellen tierischen, pflanzlichen, pilzlichen oder mikrobiellen Ursprungs gefunden und im Idealfall aus diesen isoliert werden können ("natürlich vorkommende" Stoffe).

Unter einem würzigen Geschmackseindruck versteht man insbesondere den als Umami bezeichneten Geschmack der Aminosäuren Glutaminsäure und Asparaginsäure und der Nucleotide Adenosin-5`-monophosphat, Cytidin-5'-monophosphat, Inosin-5'-monophosphat, Guanosin-5'-monophosphat, insbesondere in der Form ihrer Mononatriumsalze, insbesondere auch in Mischungen der vorgenannten Stoffe, wobei der Umami-Geschmack auch durch weitere, hier nicht aufgeführte Verbindungen verursacht werden kann. Der Geschmackseindruck Umami wird häufig mit den Begriffen "Brühe-artig", "fleischig", "mundfüllend" und "würzig" umschrieben und häufig im Zusammenhang mit dem Geschmackseindruck Kokumi gesehen. Zudem trägt der Umami-Geschmackseindruck häufig im Rahmen der Gesamtgeschmackswahrnehmung zur Salzigkeit bei, obwohl Salzigkeit insbesondere durch Natriumionen, vor allem in Form von Natriumchlorid, hervorgerufen wird. Die vorstehend genannten Aminosäuren und Nucleotide bzw. Salze haben den Nachteil, dass eine relativ hohe Konzentration dieser Stoffe eingesetzt werden muss, um einen zufriedenstellenden Umami- bzw. Kokumi-Geschmackseindruck zu vermitteln. So muss beispielsweise Natriumgluatamat regelmäßig in einer Konzentration von 0,02 bis 0,5 Gew.-%, bezogen auf das Gesamtgewicht des Lebensmittels, vorhanden sein, um den gewünschten Geschmackseindruck zu erzeugen. Die vorstehend genannten Nucleotide sind zudem für sich alleine jeweils nur schwach wirksam und können daher häufig nur mit Natriumglutamat zusammen einen zufriedenstellenden Umami-Geschmackseindruck vermitteln.

Erst in den letzten Jahren sind einige (nicht natürlich vorkommende) Verbindungen mit einer wesentlich potenteren Umami-ähnlichen Wirkung beschrieben worden, z. B. in den Schriften EP 1,989,944, WO 2008/046,895, EP 2,168,442, US2004/202,760, US 2006/057,268 und US 2007/134,389. Diese Verbindungen besitzen angeblich häufig eine 10- bis 10000-fach stärkere Geschmacksstoffwirkung als Mononatriumglutamat.

Durch molekularbiologische Methoden ist es außerdem gelungen, den wesentlichen Rezeptor des Menschen, der für den Glutamat- und Umami-Geschmack zuständig ist, zu identifizieren. Basierend auf diesen Erkenntnissen wurden in WO 2003/004,992 und WO 2003/001,876 Messmethoden vorgeschlagen, um neue möglicherweise sensorisch wirksame Umami-Geschmacksstoffe zu identifizieren. So werden in der Veröffentlichung US 2005/084506 einige potentielle Aktivatoren des Umami-Rezeptors in Form nicht natürlich vorkommender Amide beschrieben. In den Beispielen D16 (S. 120 von US 2005/084506) und 81 (S. 67 von US 2005/084506) sind nicht natürlich vorkommende Zimtamide aromatischer Amine beschrieben, die angeblich den fremdexprimierten humanen, potentiellen Umami-Rezeptor aktivieren können.

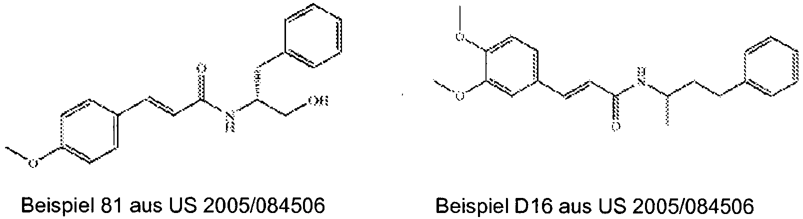

Die vorstehend genannten Verbindungen sind nicht natürlich vorkommende Verbindungen. Generell - wie auch im Rahmen der vorliegenden Erfindung - sind jedoch natürlich vorkommende Verbindungen gegenüber synthetischen bzw. nicht-natürlich vorkommenden Verbindungen bevorzugt. Natürlich vorkommende, einen potenten Umami-Geschmackseindruck vermittelnde oder verstärkende Amide vorstehend genannter Strukturtypen wurden im Stand der Technik bisher jedoch nicht beschrieben.

In der Veröffentlichung EP 1,323,356 wird zwar die Verwendung bestimmter natürlich vorkommender Zimtsäureamide aromatischer Amine als Aromastoffe beschrieben, jedoch offenbart EP 1,323,356 diesbezüglich lediglich, dass diese Verbindungen (in den darin beschriebenen Konzentrationen) als scharf oder wärmend empfunden werden, das heißt einen bestimmten chemesthetischen Reiz vermitteln. Etwaige durch die in EP 1,323,356 beschriebenen Verbindungen vermittelte (primäre) Geschmackseindrücke sind darin jedoch nicht beschrieben.

Im Ergebnis bestand die primäre Aufgabe der vorliegenden Erfindung darin, neue Verwendungen vorzugsweise natürlich vorkommender Verbindungen als Geschmacksstoffe zum Vermitteln, Modifizieren und/oder Verstärken einer, zwei oder sämtlicher der Geschmackseindrücke Umami, Kokumi und salzig bereitzustellen. Besonders bevorzugt waren Verwendungen anzugeben, wobei Verbindungen eingesetzt werden, die eine wenigstens 10-fach stärkere Geschmacksstoffwirkung als Natriumglutamat besitzen.

Eine weitere Aufgabe der vorliegenden Erfindung bestand darin, neue Geschmacksstoffmischungen anzugeben.

Im Rahmen der vorliegenden Erfindung war es besonders erstrebenswert, für die Zwecke der vorliegenden Erfindung solche Verbindungen zu identifizieren, die natürlich vorkommen und beispielsweise in Form bzw. als Bestandteil eines pflanzlichen Extrakts bereitgestellt werden können. Dementsprechend bestand eine weitere Aufgabe der vorliegenden Erfindung in der Bereitstellung solcher pflanzlicher Extrakte.

Des Weiteren galt es, neue der Ernährung oder dem Genuss dienende (gebrauchs- oder verzehrfertige) Zubereitungen und Halbfertigwaren zur Herstellung davon bereitzustellen. Natriumglutamat-reduzierte oder -freie Zubereitungen sind dabei besonders bevorzugt.

Weitere der vorliegenden Erfindung zu Grunde liegende Aufgaben ergeben sich aus der vorliegenden Beschreibung, den Bespielen sowie insbesondere den beigefügten Patentansprüchen.

Die primäre Aufgabe der vorliegenden Erfindung wird erfindungsgemäß gelöst durch die Verwendung einer Verbindung der Formel (I) 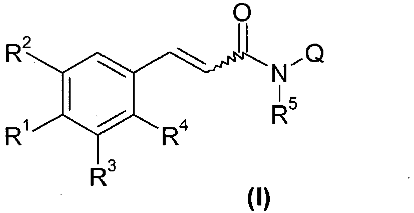 oder einer Mischung umfassend oder bestehend aus zwei oder mehr Verbindungen der Formel (I), wobei für die bzw. jede Verbindung der Formel (I) gilt:
- R¹ und R² stellen jeweils eine Methoxy-Gruppe dar
   oder
   R¹ und R² bilden zusammen eine Gruppe -O-CH₂-O-
   oder
   R¹ und R² stellen jeweils Wasserstoff dar,
- R³ und R⁴ stellen jeweils unabhängig voneinander Wasserstoff, eine Hydroxy-Gruppe oder eine Methoxy-Gruppe dar,
- R⁵ stellt Wasserstoff oder eine Methyl-Gruppe dar,
- Q stellt eine Gruppe 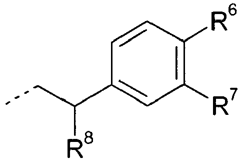 dar, wobei
   R⁶ und R⁷ jeweils eine Methoxy-Gruppe darstellen oder zusammen eine Gruppe-O-CH₂-O- bilden
   und R⁸ Wasserstoff, eine Hydroxy-Gruppe oder eine Methoxy-Gruppe darstellt, oder Q stellt eine Gruppe 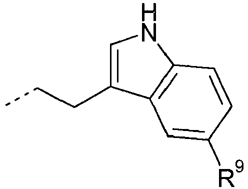
dar, wobei
R⁹ Wasserstoff, eine Hydroxy-Gruppe oder eine Methoxy-Gruppe darstellt,
als Geschmacksstoff bzw. Geschmacksstoffmischung zum Vermitteln, Modifizieren und/oder Verstärken einer, zwei oder sämtlicher der Geschmackseindrücke Umami, Kokumi und salzig.

Gemäß einer bevorzugten Ausgestaltung der erfindungsgemäßen Verwendung stellen sämtliche Reste R¹ bis R⁴ der bzw. einer, mehrerer oder sämtlicher Verbindungen der Formel (I) Wasserstoff dar.

Erfindungsgemäß besonders bevorzugt ist eine Verwendung (wie oben beschrieben), wobei für die bzw. eine, mehrere oder sämtliche Verbindungen der Formel (I) gilt:
- R¹ und R² stellen jeweils eine Methoxy-Gruppe dar
   oder
   R¹ und R² bilden zusammen eine Gruppe -O-CH₂-O-
   oder
   R¹ und R² stellen jeweils Wasserstoff dar,
- R³ und R⁴ stellen jeweils unabhängig voneinander Wasserstoff dar
- R⁵ stellt Wasserstoff oder eine Methyl-Gruppe dar,
- Q stellt eine Gruppe 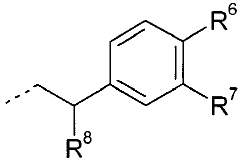 dar, wobei
   R⁶ und R⁷ jeweils eine Methoxy-Gruppe darstellen oder zusammen eine Gruppe-O-CH₂-O- bilden
   und R⁸ Wasserstoff darstellt.

Vorzugsweise ist bzw. sind die bzw. eine, mehrere oder sämtliche der erfindungsgemäß zu verwendenden Verbindungen der Formel (I) natürlich vorkommende Verbindungen, insbesondere natürlich vorkommende Zimtsäureamide aromatischer Amine.

Die im Rahmen des vorliegenden Textes beschriebenen Verbindungen der Formel (I) können als (Z)- oder (E)-Isomer oder als Mischung derselben vorliegen.

Im Falle, dass R⁸ nicht Wasserstoff ist, können die natürlich vorkommenden Zimtsäureamiden aromatischer Amine zusätzlich als (R)- oder (S)-Isomere oder als Mischung derselben vorkommen.

Erfindungsgemäß besonders bevorzugt ist eine Verwendung (wie oben beschrieben), wobei die Verbindung der Formel (I) bzw. eine, mehrere oder (vorzugsweise) sämtliche der Verbindungen der Formel (I) ausgewählt ist bzw. sind aus der Gruppe bestehend aus
Verbindung (1) der Formel 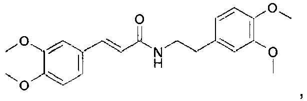 Verbindung (2) der Formel 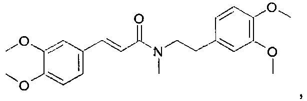 Verbindung (3) der Formel 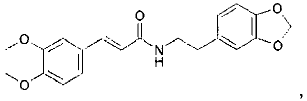 Verbindung (4) der Formel 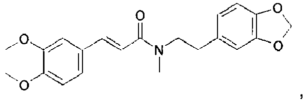 Verbindung (5) der Formel 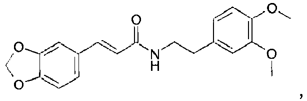 Verbindung (6) der Formel 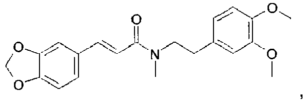 Verbindung (7) der Formel 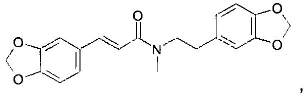 Verbindung (8) der Formel 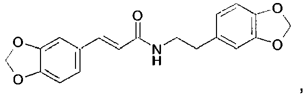 Verbindung (9) der Formel 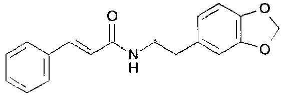 und
Verbindung (10) der Formel

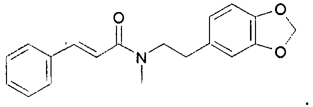

Die vorstehend genannten Verbindungen (1) bis (10), insbesondere die Verbindungen (1) und (3), sind für die Zwecke der vorliegenden Erfindung besonders geeignet.

Eine Übersicht natürlich vorkommender Zimtsäureamide aromatischer Amine ist z. B. in Kapitel 3 des Übersichtsartikels von Bassard, J.-E.; Ullmann, P.; Bernier, F.; Werck-Reichhart, D., Phytochemistry 2010, 71, (16), 1808-1824 zu finden.

Die für die Zwecke der vorliegenden Erfindung besonders bevorzugten (natürlich vorkommenden) Verbindungen (1) bis (10) sind beispielsweise aus folgenden Veröffentlichungen bekannt:

| **Verbindung** | **Struktur** | **Quelle** | **Veröffentlichung** |
|---|---|---|---|
| Verbindung (1) (Rubemamin) | 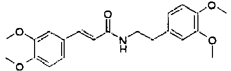 | *Zanthoxylum rubescens* (Spuren) | S.K. Adesina und J. Reisch, Phytochemistry 1989, 28 (3), 839-842 |
| | | *Chenopodium album* (5-10 mg/kg) | Cutillo, F.; D'Abrosca, B.; DellaGreca, M.; Di Marino, C.; Golino, A.; Previtera, L.; Zarrelli, A. Phytochemistry 2003, 64, 1381-1387 |
| Verbindung (2) (Rubemamid) | 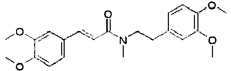 | *Zanthoxylum rubescens* | S.K. Adesina und J. Reisch, Phytochemistry 1989, 28 (3), 839-842 |
| Verbindung (3) (Rubescenamin) | 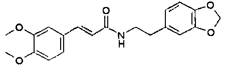 | *Zanthoxylum rubescens* | S.K. Adesina, Planta Medica 1989, 55 (3), 324-326 |
| Verbindung (4) (Rubescenamid) | 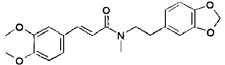 | *Zanthoxylum rubescens* | S.K. Adesina, Planta Medica 1989, 55 (3), 324-326 |
| Verbindung (5) (Zanthosin) | 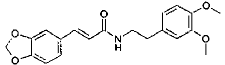 | *Zanthoxylum rubescens* | S.K. Adesina, Planta Medica 1989, 55 (3), 324-326 |
| Verbindung (6) (Zanthosinamid) | 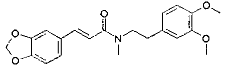 | *Zanthoxylum rubescens* | S.K. Adesina, O.A. Olatunji, D. Bergenthal und J. Reisch, Pharmazie 1988, 43 (7), 517-518 |
| Verbindung (7) (Dioxamid) | 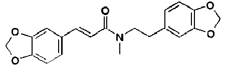 | *Zanthoxylum rubescens* | S.K. Adesina und J. Reisch, Phytochemistry 1989, 28 (3), 839-842 |
| Verbindung(8) (Dioxamin) | 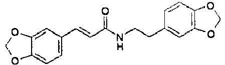 | *Zanthoxylum rubescens* | S.K. Adesina und J. Reisch, Phytochemistry 1989, 28 (3), 839-842 |
| Verbindung (9) (Zanthomamin) | 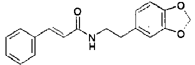 | *Zanthoxylum rubescens* | S.K. Adesina und J. Reisch, Phytochemistry 1989, 28 (3), 839-842 |
| Verbindung (10) (Zanthomamid) | 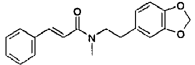 | *Zanthoxylum rubescens* | S.K. Adesina und J. Reisch, Phytochemistry 1989, 28 (3), 839-842 |

Insbesondere in Anbetracht der einleitend bereits erwähnten Veröffentlichung EP 1,323,356 war es überraschend, dass die erfindungsgemäß zu verwendenden Verbindungen der Formel (I) bzw. die erfindungsgemäß zu verwendenden Mischungen (entgegen der Erwartung gemäß EP 1,323,356) bei höheren Konzentrationen nur noch einen unterschwelligen chemesthetischen Reiz vermitteln bzw. erzeugen, dafür jedoch insbesondere in stark Natriumglutamat-reduzierten, in Natriumglutamat-freien Lebensmitteln sowie in Lebensmitteln mit reduziertem Natriumchloridgehalt (zum Beispiel in würzigen Lebensmitteln wie Tomatensuppe, Hühnersuppe, Knabbergebäck, Fertigpizza, Kartoffelchips und Popcorn) sowohl im Anfangsgeschmack (Impact) als auch in der länger anhaltenden Geschmackswahrnehmung insbesondere einen Umami-Geschmackseindruck vermitteln, modifizieren und/oder verstärken können. Die im Rahmen eigener Untersuchungen aufgefundene zusätzliche vorteilhafte Eigenschaft erfindungsgemäß zu verwendender Verbindungen der Formel (I), insbesondere erfindungsgemäß bevorzugt zu verwendender Verbindungen der Formel (I) (wie oben beschrieben), zudem einen salzigen Geschmackseindruck zu vermitteln, zu modifizieren und/oder zu verstärken (ähnlich dem Mononatriumglutamat), ermöglicht ein als besonders angenehm empfundenes Geschmackserlebnis, welches in vielen Fällen sogar als gegenüber Natriumglutamat bevorzugt bewertet wird.

Erfindungsgemäß besonders bevorzugt ist eine wie oben beschriebene Verwendung, wobei die Verbindung der Formel (I) beziehungsweise beide Verbindungen (I) bzw. eine oder zwei der Verbindungen der Formel (I) ausgewählt ist bzw. sind aus der Gruppe bestehend aus
Verbindung (1) der Formel 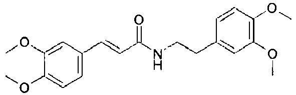 und
Verbindung (3) der Formel

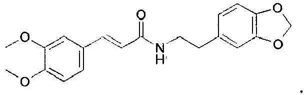

Besonders bevorzugt ist zudem die Verwendung einer Mischung (wie oben beschrieben), wobei die Mischung sowohl eine Verbindung (1) (wie oben beschrieben) als auch eine Verbindung (3) (wie oben beschrieben) umfasst oder daraus besteht. Eine im Rahmen der vorliegenden Erfindung bereitgestellte Mischung umfassend oder bestehend aus einer Verbindung (1) und einer Verbindung (3) (wie jeweils oben beschrieben) eignet sich vorteilhafterweise besonders gut zum Vermitteln, Modifizieren und/oder Verstärken einer, zwei oder sämtlicher der Geschmackseindrücke Umami, Kokumi und salzig.

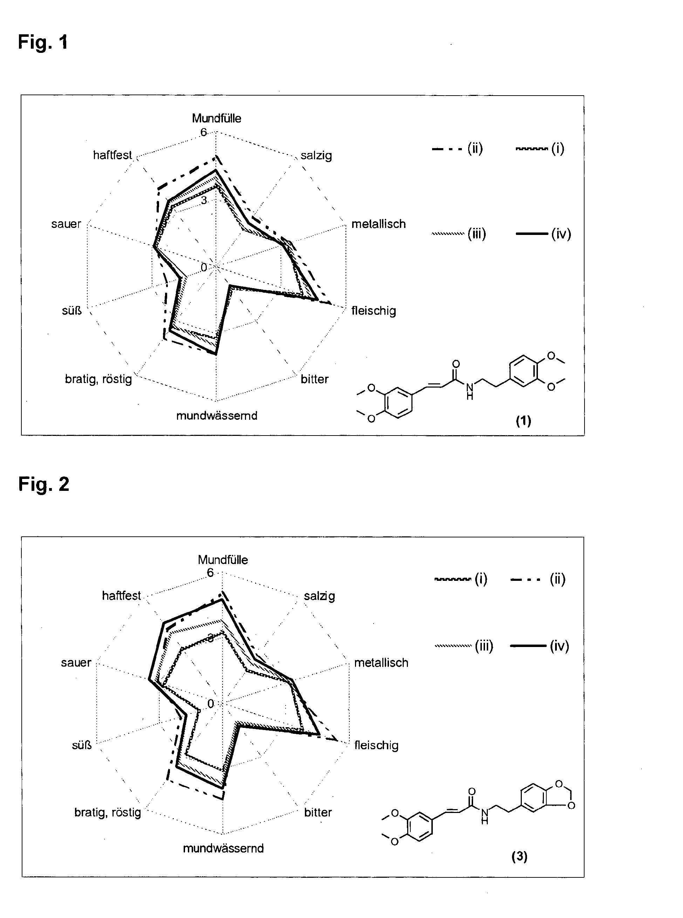

Im Vergleich zu anderen nach Umami schmeckenden Verbindungen zeichnen sich beispielsweise Rubemamin (Verbindung (1)) schon bei 10 mg/kg und Rubescenamin (Verbindung (3)) bei 5 mg/kg Testkonzentration durch einen dem Natriumglutamat (MSG) sehr nahekommenden Umami-Geschmack aus, welcher vor allem die Mundfülle und den fleischigen Charakter sowie den mundwässernden Effekt deutlich verstärkt, ohne dabei unangenehm süß zu schmecken. Dies zeigt auch das als Figur 1 beigefügte Netzdiagramm aus Beispiel 2, in welchem ein amerikanischer Rindfleischextrakt als Base mit (i) einer solchen Base mit einem Zusatz von 10 ppm (iii) bzw. 50 ppm Rubemamin (Verbindung (1)) (iv) und mit einer Probe (ii) einer solchen Base mit einem Zusatz von 0,05 Gew.-% MSG (Natriumglutamat) verglichen wird. Verbindung (3) zeigt in einem analogen Experiment (dargestellt in Figur 2 aus Beispiel 2) schon bei 5 ppm eine starke Wirkung (Kurve iii) und kann bei 10 ppm (Kurve iv) für die meisten Deskriptoren eine ähnlich starke Bewertung wie die Probe (ii) einer solchen Base mit einem Zusatz von 0,05 Gew.-% MSG erzielen. Die vorstehenden Beobachtungen gelten tendenziell entsprechend für alle erfindungsgemäß zu verwendenden Verbindungen bzw. Mischungen.

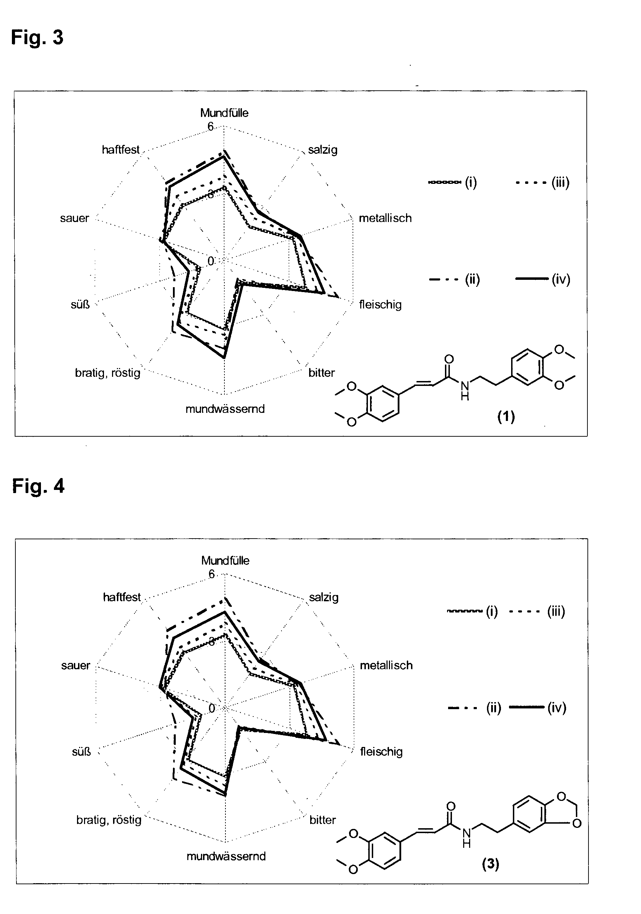

Eine Verstärkung des Natriumglutamat-Effekts wird weiterhin durch das als Figur 3 aus Beispiel 3 beigefügte Netzdiagramm belegt. Hier wird (i) ein Rindfleischextrakt als Base mit (ii) einer solchen Base unter Zusatz von 0,05 % Natriumglutamat, (iii) einer solchen Base unter Zusatz von 0,0025 % Natriumglutamat und (iv) einer solchen Base unter Zusatz von 0,0025 % Natriumglutamat und 20 ppm Rubemamin (Verbindung (1)) verglichen. Während die Probe (iii) insgesamt hinsichtlich der abgefragten Parametern nur wenig stärker als die Probe (i) ohne Zusatz wahrgenommen wurde, sind die stark Glutamat-haltige Probe (ii) und die Probe mit geringer Konzentration Glutamat und Rubemamin (iv) bis auf eine etwas geringere Süße kaum unterscheidbar.

Rubescenamin (Verbindung (3)) ist vorteilhafterweise auch in besonders geringer Konzentration in der Lage, den Natriumglutamat-Effekt zu verstärken, wie durch das als Figur 4 beigefügte Netzdiagramm aus Beispiel 3 belegt werden kann. Hier wird wiederum (i) ein Rindfleischextrakt als Base mit (ii) einer solchen Base unter Zusatz von 0,05 % Natriumglutamat, (iii) einer solchen Base unter Zusatz von 0,0025 % Natriumglutamat und (iv) einer solchen Base unter Zusatz von 0,0025 % Natriumglutamat und mit einem Zusatz von 5 ppm Rubescenamin (Verbindung (3)) verglichen. Auch hier wurde die Probe mit geringerer Konzentration an Glutamat und Rubescenamin (iv) hinsichtlich nahezu aller Parameter stärker als Probe (iii), und insbesondere hinsichtlich des Parameters "salzig" ähnlich der Probe (ii) bewertet.

Im Rahmen der vorliegenden Erfindung wird auch ein Verfahren zum Vermitteln, Modifizieren und/oder Verstärken eines Geschmackseindrucks ausgewählt aus der Gruppe bestehend aus Umami, Kokumi und salzig, beschrieben. Dabei wird einer bereitgestellten Substanz oder Zusammensetzung eine erfindungsgemäß zu verwendende Verbindung der Formel (I) bzw. eine erfindungsgemäß zu verwendende Mischung (wie jeweils oben beschrieben) oder eine erfindungsgemäße Geschmacksstoffmischung (s. hierzu weiter unten) in einer Umami-geschmacklich wirksamen Menge zugegeben. Für bevorzugt einzusetzende Verbindungen der Formel (I) bzw. Mischungen und erfindungsgemäße Geschmacksstoffmischungen gilt hierbei das im Rahmen des vorliegenden Textes Gesagte entsprechend.

Die vorliegende Erfindung betrifft insbesondere erfindungsgemäße Verwendungen (wie oben beschrieben) in einer der Ernährung oder dem Genuss dienenden Zubereitung. Bevorzugt Zubereitungen sind weiter unten beschrieben.

Ein weiterer Aspekt der vorliegenden Erfindung betrifft neue Geschmacksstoffmischungen.

Eine erfindungsgemäße Geschmacksstoffmischung umfasst oder besteht aus
(i) einer Verbindung der Formel (I) oder einer Mischung umfassend oder bestehend aus zwei oder mehr Verbindungen der Formel (I), wie jeweils in oben definiert, vorzugsweise wie oben als bevorzugt bezeichnet,
   sowie
(ii) einem, zwei, drei oder mehreren weiteren Geschmacksstoffen zum Vermitteln, Modifizieren und/oder Verstärken einer, zwei oder sämtlicher der Geschmackseindrücke Umami, Kokumi und salzig, wobei der bzw. die weiteren Geschmacksstoffe keine Verbindungen der Formel (I) sind.

Im Rahmen der vorliegenden Erfindung gilt generell, dass die erfindungsgemäß zu verwendenden Verbindungen der Formel (I) bzw. Mischungen davon in erfindungsgemäßen Verwendungen oder als Bestandteil erfindungsgemäßer Geschmacksstoffmischungen, Zubereitungen oder Halbfertigwaren vorzugsweise in Kombination mit einem, zwei, drei oder mehreren weiteren Geschmacksstoffen zum Vermitteln, Modifizieren und/oder Verstärken einer, zwei oder sämtlicher der Geschmackseindrücke Umami, Kokumi und salzig, eingesetzt werden, wobei diese weiteren Geschmacksstoffe keine Verbindungen der Formel (I) sind.

Des Weiteren werden die erfindungsgemäß zu verwendenden Verbindungen der Formel (I) bzw. Mischungen davon im Rahmen der vorliegenden Erfindung vorzugsweise in Kombination mit einer oder mehreren, nicht der Formel (I) entsprechenden Substanz(en) zum Maskieren oder Vermindern eines unangenehmen Geschmackseindrucks (insbesondere eines bitteren, metallischen, kalkigen, sauren und/oder adstringierenden Geschmackseindrucks) und/oder zur Verstärkung oder zum Vermitteln eines (weiteren) angenehmen Geschmackseindrucks (z. B. süß) eingesetzt.

Besonders bevorzugt ist der bzw. sind zwei, drei, mehrere oder sämtliche der weiteren Geschmacksstoffe zum Vermitteln, Modifizieren und/oder Verstärken der Geschmackseindrücke Umami, Kokumi und/oder salzig, die erfindungsgemäß mit den Verbindungen der Formel (I) oder Mischungen davon kombiniert werden (wie oben beschrieben), vorzugsweise ausgewählt aus der Gruppe bestehend aus Mononatriumglutamat, freie Glutaminsäure, Nucleotide oder deren pharmazeutisch akzeptable Salze, Strombine, Theogalline, Pyridin-Betain-Verbindungen, Glutaminsäureglycoside, Äpfelsäureglycoside, Glutathion-Derivate, Lactisole und Alkylpyridine (vorzugsweise Alkylpyridine wie in WO2009 122318 und WO2009 1223319 beschrieben), insbesondere 2-Hexyl-, 2-Heptyl und 2-Octylpyridin, (2E,6Z)-N-cyclopropylnona-2,6-dienamid, (2E,6Z)-N-ethylnona-2,6-dienamid, N-[(2E)-3,7-dimethylocta-2,6-dienyl]cyclopropancarboxamid, N'-[(2-methoxy-4-methyl-phenyl)methyl]-N-[2-(5-methyl-2-pyridyl)ethyl]oxamid, N'-[(2,4-dimethoxyphenyl)methyl]-N-[2-(2-pyridyl)ethyl]oxamid, N'-[(2-methoxy-4-methyl-phenyl)methyl]-N-[2-(2-pyridyl)ethyl]oxamid, N-(1-propylbutyl)-1,3-benzodioxole-5-carboxamid, 1-(2-hydroxy-4-isobutoxy-phenyl)-3-(2-pyridyl)propan-1-on und 1-(2-hydroxy-4-methoxy-phenyl)-3-(2-pyridyl)propan-1-on.

Generell ist der bzw. sind zwei, drei, mehrere oder sämtliche der weiteren Geschmacksstoffe zum Vermitteln, Modifizieren und/oder Verstärken einer, zwei oder sämtlicher der Geschmackseindrücke Umami, Kokumi und salzig vorzugsweise natürlich vorkommende Verbindungen, besonders bevorzugt Verbindungen ausgewählt aus der Gruppe bestehend aus: Mononatriumglutamat, freie Glutaminsäure, Nucleotide (z.B. Adenosin-5'-monophosphat, Cytidin-5'-monophosphat, Inosin-5'-monophosphat, Guanosin-5'-monophosphat), oder deren pharmazeutisch akzeptable Salze, Strombine wie in WO 2010 100,589 beschrieben, Theogalline wie in JP 2007 110,988 beschrieben, Pyridin-Betain-Verbindungen wie in EP 1,291,342 beschrieben, Glutaminsäureglycoside wie in WO 2002 087,361 beschrieben, Äpfelsäureglycoside wie in WO 2006 003,107 beschrieben, Glutathion-Derivate wie in EP 181,421 oder WO 2007 042,273 beschrieben, Lactisole, Hydroxyflavanone (z.B. Eriodictyol, Homoeriodictyol oder deren Natriumsalze), insbesondere gemäß EP 1 258 200, Hesperitin nach EP 1,909,599, Phloretin nach EP 1,972,203 oder EP 1,998,636, Hydroxyflavane nach US 2010 292,175, 4-Hydroxychalcone nach EP 1 972 203, Extrakte auf Basis von Hydrangea dulcis gemäß EP 2,298,084, oder Rubus ssp. gemäß US Provisional Application 61/333,435 und damit verbundenen Veröffentlichungen; Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shütake), Meeralgen und Mineralsalzmischungen, insbesondere Mineralsalzmischungen nach US 2009 214,728 und damit verbundenen Veröffentlichungen.

Synthetische vorzugsweise mit erfindungsgemäß zu verwendenden Verbindungen der Formel (I) bzw. Mischungen davon zu kombinierende Geschmacksstoffe zum Vermitteln, Modifizieren und/oder Verstärken einer, zwei oder sämtlicher der Geschmackseindrücke Umami, Kokumi und salzig sind vorzugsweise ausgewählt aus den chemischen Strukturen, die in den Veröffentlichungen US 2004 0202619, US 2004 0202760, US 2006 0057268 und US 2006 0068071 beschrieben sind, insbesondere (2E, 6Z)-N-cyclopropylnona-2,6-dienamid (FEMA 4087; Flavis 16.093), 2E,6Z)-N-ethylnona-2,6-dienamid (FEMA 4113; Flavis 16.094) und N-[(2E)-3,7-dimethylocta-2,6-dienyl]cyclopropancarboxamid (FEMA 4267; Flavis 16.095), den chemischen Strukturen wie in US2005 0084506 beschrieben, insbesondere N'-[(2-methoxy-4-methylphenyl)methyl]-N-[2-(5-methyl-2-pyridyl)ethyl]oxamid (FEMA 4234; Flavis 16.190), N'-[(2,4-dimethoxyphenyl)methyl]-N-[2-(2-pyridyl)ethyl]oxamid (FEMA 4233; Flavis 16.099), N'-[(2-methoxy-4-methyl-phenyl)methyl]-N-[2-(2-pyridyl)ethyl]oxamid (FEMA 4231; Flavis 16.101), N-(1-propylbutyl)-1,3-benzodioxole-5-carboxamid (FEMA 4232; Flavis 16.098), und den chemischen Strukturen wie in WO/2011/004016 beschrieben, insbesondere 1-(2-hydroxy-4-isobutoxy-phenyl)-3-(2-pyridyl)propan-1-on (FEMA 4722) und 1-(2-hydroxy-4-methoxy-phenyl)-3-(2-pyridyl)propan-1-on (FEMA 4723).

In einer bevorzugten Ausgestaltung der vorliegenden Erfindung werden die erfindungsgemäß zu verwendenden Verbindungen der Formel (I) bzw. Mischungen davon im Rahmen der erfindungsgemäßen Verwendung bzw. in erfindungsgemäßen Geschmacksstoffmischungen, erfindungsgemäßen Zubereitungen oder erfindungsgemäßen Halbfertigwaren in Kombination mit einem oder mehreren süßverstärkenden Stoffen eingesetzt, insbesondere mit einer oder mehreren Verbindungen gemäß WO2007/014879 A1, WO 2007/107596 A1, US 2010 292,175 und EP 1 955 601, insbesondere zusammen mit Hesperetin, 3,7'-Dihydroxy-4'-methoxyflavan und/oder Phloretin. Hierdurch wird vorteilhafterweise eine Verstärkung und eine Vertiefung sowie Abrundung des Geschmacksprofils erzielt, insbesondere in erfindungsgemäßen Zubereitungen und Halbfertigwaren mit würzigem und/oder salzigem Geschmack. Der Gesamtanteil an Hesperetin, 3,7'-Dihydroxy-4'-methoxyflavan und/oder Phloretin in solchen Zusammensetzungen oder Zubereitungen liegt vorzugsweise (jeweils) im Bereich von 1 bis 400 ppm, bevorzugt im Bereich von 5 bis 200 ppm, bezogen auf das Gesamtgewicht der Zubereitung bzw. Halbfertigware.

Weitere vorteilhafterweise mit erfindungsgemäß zu verwendenden Verbindungen der Formel (I) bzw. Mischungen zu kombinierende Geschmacksstoffe und weitere Substanzen sind weiter unten beschrieben.

Die Gesamtmenge an Verbindungen der Formel (I) in einer erfindungsgemäßen Geschmacksstoffmischung (wie oben beschrieben) liegt vorzugsweise im Bereich von 0,0001 bis 95 Gew.-%, bezogen auf das Gesamtgewicht der Geschmacksstoffmischung, besonders bevorzugt im Bereich von 0,001 bis 95 Gew.-%.

Natürlich vorkommende Zimtsäureamide aromatischer Amine der Formel (I) sind in den natürlichen (pflanzlichen) Quellen häufig nur in äußerst geringen Mengen vorhanden. So wurde zum Beispiel Rubemamin in ganzen Pflanzen von *Chenopodium album* (weißer Gänsefuß) in 5-10 mg/kg und in der Rinde von *Zanthoxylum rubescens* lediglich in noch geringeren Spuren gefunden. Dadurch kann durch Verwendung der nativen oder getrockneten Pflanzenteile oder zum Teil auch der einfachen, nicht weiter aufgereinigten Extrakte in verzehrfertigen Lebensmitteln häufig kein zufriedenstellender Umami-Effekt erreicht werden. Ein Aspekt der vorliegenden Erfindung bestand daher auch darin, natürlich vorkommende Zimtsäureamide aromatischer Amine der Formel (I) in einer ausreichenden Anreicherung und Reinheit durch klassische Synthese, biomimetische/biotechnologische Synthese oder Extraktion und Aufreinigung sowie gegebenenfalls Anreicherung verfügbar zu machen, um diese in Halbfertigwaren oder verzehrfertigen der Ernährung oder dem Genuss dienenden Zubereitungen in ausreichender Konzentration (zur Erzielung des Umami-Geschmacks) einzusetzen.

Ein bevorzugter Aspekt der vorliegenden Erfindung betrifft einen pflanzlichen Extrakt umfassend oder bestehend aus
- einer Verbindung der Formel (I) oder einer Mischung umfassend oder bestehend aus zwei oder mehr Verbindungen der Formel (I), wie jeweils oben definiert, vorzugsweise wie oben als bevorzugt bezeichnet,
   sowie
- einem oder mehreren weiteren Bestandteilen,
wobei die Gesamtmenge an Verbindungen der Formel (I) bezogen auf das Gesamtgewicht des pflanzlichen Extrakts im Bereich von 1.000 bis 500.000 ppm, vorzugsweise im Bereich von 10.000 bis 100.000 ppm liegt.

Die erfindungsgemäß zu verwendenden Verbindungen der Formel (I) sind vorzugsweise durch Extraktion aus Pflanzen oder Teilen davon und optional durch übliche Verfahren angereicherte, natürlich vorkommende Verbindungen, wobei die Verbindungen besonders bevorzugt solche Verbindungen der Formel (I) sind, die aus einer Pflanze (bzw. Teilen davon) ausgewählt aus der Gruppe bestehend aus *Zanthoxylum rubescens* und *Chenopodium album* extrahiert sind.

Ein erfindungsgemäßer pflanzlicher Extrakt (wie oben beschrieben) ist dementsprechend gemäß einem bevorzugten Aspekt der vorliegenden Erfindung ein Extrakt aus *Zanthoxylum rubescens* oder *Chenopodium album.*

Dabei umfasst ein solcher Extrakt vorzugsweise nur solche weiteren Bestandteile, die bei einer Temperatur von 20°C unter Normaldruck als Feststoffe vorliegen und weiter bevorzugt keine reinen Aminosäuren, insbesondere keine Aminosäuren, die einen Umami-Geschmackseindruck vermitteln, wie z. B. Glutaminsäure und Aspariginsäure, sind.

Besonders bevorzugt sind die weiteren Bestandteile eines erfindungsgemäßen Extrakts ausschließlich Verbindungen, die mittels Ethanol oder Methanol oder eines Methanol-Ethanol-, Methanol-Wasser-, Methanol-Ethanol-Wasser- oder Ethanol-WasserGemisches, superkritischem Kohlendioxid, Essigsäureethylester, *tert*-Butylmethylether, Dichlormethan, *n*-Heptan, *n*-Hexan oder Mischungen davon aus *Zanthoxylum rubescens* oder *Chenopodium album* extrahiert werden können. Die Extraktion erfolgt dabei vorzugsweise bei Temperaturen im Bereich von -80°C bis zur Siedetemperatur des Extraktionsmittels, vorzugsweise für fünf Minuten bis 24 Stunden durch einfaches Rühren, Perkolieren oder Gegenstromextraktion. Die Extraktion insbesondere mit wasserhaltigen Gemischen kann auch in Gegenwart von pH-regulierenden Säuren, Basen oder Puffergemischen erfolgen.

Die erfindungsgemäßen Extrakte, insbesondere solche aus *Zanthoxylum rubescens* oder *Chenopodium album,* werden vorzugsweise hergestellt, indem zunächst die Pflanzenteile, vorzugsweise frische oder getrocknete oberirdische Teile (besonders bevorzugt Blätter, Stängel, Blüten, Rinde, Bast, Holz und/oder Früchte) in getrockneter und zerkleinerter Form folgenden Schritten unterworfen werden:
a) Mit einem geeigneten Lösungsmittel (z.B. Wasser, Wasser-Ethanol-Gemische, Ethanol, Methanol, 1-Propanol, 2-Propanol, Glycerin, Propan-1,2-diol, superkritisches Kohlendioxid, Essigsäureethylester, tert-Butylmethylether, Dichlormethan, n-Heptan, n-Hexan oder deren Mischungen davon) wird von 0°C bis zur Siedetemperatur des jeweiligen Lösungsmittels z.B. durch Ausrühren, Soxhlet-, Gegenstrom-, Perkolations- oder einfaches Siebkorbverfahren ein Primärextrakt extrahiert, wobei die Lösungsmittel jeweils alleine, in binären oder ternären Mischungen oder auch konsekutiv in einer auf- oder absteigenden Polaritätsfolge verwendet werden können.
   Bevorzugt ist ein konsekutives Extraktionsverfahren mit einer aufsteigenden Polaritätsfolge der Lösungsmittel. Dabei werden beginnend mit einem unpolaren Lösungsmittel die zu extrahierenden Pflanzenteile einer Extraktion unterworfen. Das Lösungsmittel wird abgezogen und der Primärextrakt dieses Extraktionsschrittes gewonnen. Nachfolgend wird wenigstens in einem weiteren Schritt die Extraktion der bereits dem ersten Extraktionsschritt unterworfenen Pflanzenteile wiederholt, wobei ein Lösungsmittel höherer Polarität eingesetzt wird und ein entsprechender Primärextrakt aus dieser Extraktionsstufe gewonnen wird.
   Die konsekutive Extraktion kann dabei mehrere Extraktionsschritte umfassen, wobei bevorzugt ist, dass der letzte Schritt durch Extraktion mit Wasser, Ethanol, Methanol, 1-Propanol, 2-Propanol, Glycerin, Propan-1,2-diol oder einer Mischung aus einem der vorgenannten nicht-wässrigen Lösungsmittel mit Wasser erfolgt. Ein in erfindungsgemäßen (oral konsumierbaren) Zubereitungen eingesetzter Extrakt wird vorzugsweise aus einem Primärextrakt des letzten Extraktionsschrittes gewonnen.
   Die Extraktionen können jeweils bei Temperaturen von -80 °C bis zur jeweiligen Siedetemperatur des Extraktionsmittels für vorzugsweise 5 Minuten bis 24 h durch einfaches Rühren, Perkolieren oder Gegenstromextraktion erfolgen.
b) Der jeweilige Primärextrakt wird gegebenenfalls durch destillative oder andere evaporative oder pervaporative Verfahren aufkonzentriert, gegebenenfalls bis nur noch Feststoffe oder wenig flüchtige Flüssigkeiten vorliegen.
c) Optional wird der (gegebenenfalls aufkonzentrierte) Primärextrakt durch Behandlung mit oder an Adsorbentien (z.B. Kieselgel, modifizierte Kieselgele (z.B. RP-Phasen), Aktivkohle, Zeolithe, Bentonit, Kieselgur, Alumina, basische oder saure oder neutrale [makroporöse] Ionentauscher) im Batch- oder Säulenchromatographieverfahren, gegebenenfalls auch unter Zuhilfenahme weiterer Lösungsmittel, bevorzugt Wasser, n-Hexan, Dichlormethan, Ameisensäure, Methanol, Ethanol, 1,2-Propylenglycol, aufgereinigt (Sekundärextrakt).
d) Optional wird der Sekundärextrakt durch destillative oder andere evaporative oder pervaporative Verfahren getrocknet (Trockenextrakt).
e) Optional wird der getrocknete Sekundärextrakt wieder in ein geeignetes Lösungsmittel oder Gemisch aufgenommen (z.B. Ethanol, 1,2-Propylenglycol, Pflanzenöltriglyceride, Triacetin oder Glycerin).

Evaporative oder pervaporative Verfahren für die Zwecke der vorliegenden Erfindung sind vorzugsweise Destillation, Sublimation, Wasserdampfdestillation, Gefriertrocknung, pervaporative Membranverfahren, Sprühtrocknung, wobei dazu vor der jeweiligen Behandlung geeignete Hilfs- und Trägerstoffe zugesetzt werden können.

Der Primärextrakt (vorzugsweise aus *Zanthoxylum rubescens* oder *Chenopodium album*) nach b) (s. oben) kann auch noch weiter aufgeschlossen werden, z.B. durch enzymatische Behandlung (z.B. mit Cellulasen zum Aufschluss der Zellen), durch Behandlung mit Säure (z.B. unter Druck), durch Behandlung mit geeigneten basischen Lösungen (z.B. von Hydroxiden, Carbonaten oder Hydrogencarbonaten von Natrium, Kalium, Calcium, Magnesium und Zink), mit sauren Ionentauschern oder mit Wasserdampf, vorzugsweise bei Drücken von 0,01 mbar bis 100 bar, besonders bevorzugt bei 1 mbar bis 20 bar.

Der Sekundärextrakt (vorzugsweise aus *Zanthoxylum rubescens* oder *Chenopodium album*) nach c) kann auch mit einem Anteil von 1 bis 99 Gew.-%, bezogen auf den getrockneten Sekundärextrakt, an Hilfs- und Trägerstoffen (z.B. Maltodextrin, Stärke, natürliche oder künstliche Polysaccharide und/oder Pflanzengummen wie modifizierte Stärken oder Gummi Arabicum) versetzt werden, um die Herstellung eines Trockenextrakts nach d) zu verbessern.

Im Rahmen der vorliegenden Erfindung bevorzugte, für Nahrungs- und Genussmittel zur Extraktion geeignete Lösungsmittel sind Wasser, Ethanol, Methanol, 1-Propanol, 2-Propanol, Propan-1,2-diol, Glycerin, Aceton, Dichlormethan, Essigsäureethylester, Diethylether, Hexan, Heptan, Triacetin, pflanzliche Öle oder Fette, superkritisches Kohlendioxid und Mischungen davon. Besonders bevorzugt sind Wasser, Ethanol, Methanol, 1-Propanol, 2-Propanol, Glycerin, Propan-1,2-diol Mischungen davon, insbesondere Mischungen eines oder mehrerer der vorgenannten nicht-wässrigen Lösungsmittel mit Wasser, insbesondere zur Verwendung in einem Extraktionsschritt, der wenigstens einem Extraktionsschritt mit einem weniger polaren (bevorzugt unpolaren) Lösungsmittel folgt. Es hat sich nämlich überraschenderweise gezeigt, dass auf diese Art der konsekutiven Extraktion im Extrakt des insbesondere letzten Extraktionsschrittes eine Vielzahl von oder sämtliche der störenden Geschmacksnoten nicht mehr vorhanden sind. Bevorzugte Hilfs- oder Trägerstoffe sind Maltodextrin, Stärke, natürliche oder künstliche Polysaccharide und/oder Pflanzengummen wie modifizierte Stärken oder Gummi Arabicum, für die Aromakompositionen zugelassene Lösungsmittel wie z.B. Ethanol, 1,2-Propylenglycol, Wasser, Glycerin, Triacetin, Pflanzenöltriglyceride, färbende Mittel, z.B. zugelassene Lebensmittelfarbstoffe, färbende Pflanzenextrakte, Stabilisitoren, Konservierungsstoffe, Antioxidantien und Viskosität-beeinflussende Stoffe.

Der aufkonzentrierte bzw. trockene Primärextrakt (vorzugsweise aus *Zanthoxylum rubescens* oder *Chenopodium album*) nach b) enthält vorzugsweise 0,001 bis 80 Gew.-% bevorzugt 0,005 bis 50 Gew.-%, besonders bevorzugt 0,01 bis 25 Gew.-%, weiter bevorzugt 0,1 bis 25 Gew.-% an Verbindungen der Formel (I), jeweils bezogen auf das Trockengewicht des aufkonzentrierten Primärextrakts.

Der aufkonzentrierte bzw. trockene Sekundärextrakt (vorzugsweise aus *Zanthoxylum rubescens* oder *Chenopodium album*) nach d) enthält vorzugsweise 0,001 bis 100 Gew.-% bevorzugt 1 bis 99 Gew.-%, besonders bevorzugt 10 bis 95 Gew.-% der Verbindungen der Formel (I), jeweils bezogen auf das Trockengewicht des aufkonzentrierten Sekundärextraktes.

Die vorliegende Erfindung betrifft auch die Verwendung eines erfindungsgemäßen pflanzlichen Extraktes (wie oben beschrieben) zum Vermitteln, Modifizieren und/oder Verstärken einer, zwei oder sämtlicher der Geschmackseindrücke Umami, Kokumi und salzig.

Die erfindungsgemäß zu verwendenden Verbindungen der Formel (I) können auch durch (bekannte) synthetische präparative Methoden (z. B. aus den entsprechenden Säuren mit den Aminen, wie z. B. beschrieben in DE-19,737,327) erhalten werden oder durch enzymatische (z. B. wie beschrieben in WO2004 033,699) oder fermentative Prozesse gewonnen werden, die auch in Pflanzen (z.B. Negrel, J.; Javelle, F.; Paynot, M., Woundinduced tyramine hydroxycinnamoyl transferase in potato (Solanum tuberosum) tuber disks. J. Plant Physiol. 1993, 142, (5), 518-524) oder Ishihara, A.; Kawata, N.; Matsukawa, T.; Iwamura, H., Induction of N-Hydroxycinnamoyltyramine synthesis and tyramine N-Hydroxycinnamoyltransferase (THT) activity by wounding in maize leaves, Biosci. Biotechnol. Biochem. 2000, 64, (5), 1025-1031) oder Pflanzenzellkulturen durchgeführt werden können.

Wie einleitend erwähnt, betrifft die vorliegende Erfindung auch der Ernährung oder dem Genuss dienende gebrauchs- oder verzehrfertige Zubereitungen. Eine erfindungsgemäße Zubereitung umfasst oder besteht gemäß einer ersten Alternative aus
(a1) einer Verbindung der Formel (I) oder einer Mischung umfassend oder bestehend aus zwei oder mehr Verbindungen der Formel (I), wie oben definiert, vorzugsweise wie oben als bevorzugt bezeichnet,
   sowie
(b) einem oder mehreren weiteren zum Verzehr geeigneten Bestandteilen, wobei der bzw. die weiteren Bestandteile keine Verbindungen der Formel (I) sind,
wobei die Gesamtmenge an Verbindungen der Formel (I) bezogen auf das Gesamtgewicht der gebrauchs- oder verzehrfertigen Zubereitung im Bereich von 5 ppm bis 500 ppm, bevorzugt im Bereich von 5 ppm bis 200 ppm, insbesondere bevorzugt im Bereich von 5 ppm bis 100 ppm liegt.

Besonders bevorzugt ist es, wenn die Gesamtmenge an Verbindungen der Formel (I) bezogen auf das Gesamtgewicht der gebrauchs- oder verzehrfertigen Zubereitung im Bereich von 10 bis 200 ppm, besonders bevorzugt im Bereich von 25 bis 100 ppm liegt.

Gemäß einer zweiten Alternative umfasst oder besteht eine erfindungsgemäße Zubereitung aus
(a2) einer Geschmackstoffmischung wie oben beschrieben bzw. einem pflanzlichen Extrakt wie oben beschrieben sowie
(b) einem oder mehreren weiteren zum Verzehr geeigneten Bestandteilen, wobei der bzw. die weiteren Bestandteile keine Verbindungen der Formel (I) sind.

Für bevorzugte Verbindungen der Formel (I) bzw. Mischungen sowie für bevorzugte Ausgestaltungen der einzusetzenden Geschmacksstoffmischungen oder des einzusetzenden pflanzlichen Extrakts gilt dabei jeweils das oben Gesagte entsprechend.

Vorzugsweise liegt dabei die Gesamtmenge an Verbindungen der Formel (I) bezogen auf das Gesamtgewicht der Zubereitung im Bereich von 1 ppm bis 500 ppm, bevorzugt im Bereich von 1 ppm bis 200 ppm, besonders bevorzugt im Bereich von 1 ppm bis 100 ppm.

Vorzugsweise gilt auch für eine erfindungsgemäße Zubereitung gemäß vorstehend beschriebener zweiter Alternative, dass die Gesamtmenge an Verbindungen der Formel (I) bezogen auf das Gesamtgewicht der Zubereitung besonders bevorzugt im Bereich von 5 ppm bis 500 ppm, weiter bevorzugt im Bereich von 5 ppm bis 200 ppm, besonders bevorzugt im Bereich von 5 ppm bis 100 ppm liegt.

Besonders bevorzugt ist es, wenn die Gesamtmenge an Verbindungen der Formel (I) bezogen auf das Gesamtgewicht der gebrauchs- oder verzehrfertigen Zubereitung im Bereich von 10 bis 200 ppm, besonders bevorzugt im Bereich von 25 bis 100 ppm liegt.

Die erfindungsgemäßen gebrauchs- oder verzehrfertigen (der Ernährung oder dem Genuss dienenden) Zubereitungen sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel, siehe auch weiter unten) oder wieder aus der Mundhöhle entfernt zu werden (z.B. Kaugummis). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen aufgenommen zu werden. Hierzu zählen auch Stoffe, die Lebensmitteln bei ihrer Herstellung, Verarbeitung oder Bearbeitung zugesetzt werden und dazu vorgesehen sind, in die menschliche Mundhöhle eingebracht zu werden.

Im Rahmen des vorliegenden Textes werden unter einem "Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

Die vorliegende Erfindung betrifft auch erfindungsgemäße Halbfertigwaren zur Herstellung einer der Ernährung oder dem Genuss dienenden Zubereitung (vorzugsweise einer erfindungsgemäßen Zubereitung) umfassend oder bestehend aus
(a) einer Verbindung der Formel (I) oder einer Mischung umfassend oder bestehend aus zwei oder mehr Verbindungen der Formel (I), wie oben definiert, vorzugsweise wie oben als bevorzugt bezeichnet, oder
   einer Geschmackstoffmischung wie oben beschrieben
   oder
   einem pflanzlichen Extrakt wie oben beschrieben
   sowie
(b) einem oder mehreren weiteren zum Verzehr geeigneten Bestandteilen, wobei der bzw. die weiteren Bestandteile keine Verbindungen der Formel (I) sind,
wobei die Gesamtmenge an Verbindungen der Formel (I) bezogen auf das Gesamtgewicht der Halbfertigware im Bereich von 10 ppm bis 500.000 ppm, bevorzugt im Bereich von 25 ppm bis 100.000 ppm, insbesondere im Bereich von 50 ppm bis 15.000 ppm, liegt.

Unter einer Halbfertigware ist im Zusammenhang des vorliegenden Textes ein Produkt zu verstehen, welches aufgrund eines sehr hohen Gehaltes an Aroma- und Geschmacksstoffen für die Verwendung als verzehrfertiges Lebensmittel ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges Lebensmittel überführt. Als Beispiel für Halbfertigwaren seien hier Tütensuppen, Backaromen und Puddingpulver genannt.

Eine Reihe von erfindungsgemäßen Zubereitungen oder Halbfertigwaren (wie oben beschrieben) ist besonders bevorzugt. So sind beispielsweise erfindungsgemäße Zubereitungen oder Halbfertigwaren gemäß einer bevorzugten Ausführungsform sprühgetrocknete Zubereitungen bzw. Halbfertigwaren, die als weitere zum Verzehr geeignete Bestandteile (unter anderem) feste Trägerstoffe umfassen.

Erfindungsgemäß bevorzugt sind erfindungsgemäße Zubereitungen oder Halbfertigwaren, die einen oder mehrere zum Verzehr geeignete feste Trägerstoffe umfassen, wobei, bezogen auf die Trockenmasse der Zubereitung bzw. Halbfertigware, das Gewichtsverhältnis der Gesamtmenge an Verbindungen der Formel (I) zu der Gesamtmenge an zum Verzehr geeigneten festen Trägerstoffen vorzugsweise im Bereich von 1 : 10 bis 1 : 100000, bevorzugt im Bereich von 1 : 50 (vorzugsweise 1 : 100) bis 1 : 20000, besonders bevorzugt im Bereich von 1 : 100 (vorzugsweise 1 : 1000) bis 1 : 5000, liegt.

Weiter bevorzugt ist es, wenn dabei die Gesamtmenge von Verbindungen der Formel (I) und zum Verzehr geeigneten festen Trägerstoffen bezogen auf das Gesamtgewicht der Zubereitung bzw. Halbfertigware im Bereich von 70 bis 100 Gew.-%, bevorzugt im Bereich von 85 bis 100 Gew.-%, liegt.

Vorteilhafte Trägerstoffe sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum. Bevorzugte Stärkehydrolysate sind Maltodextrine und Dextrine.

Erfindungsgemäß besonders bevorzugte Trägerstoffe sind Siliciumdioxid, Gummi Arabicum und Maltodextrine, wobei hier wiederum Maltodextrine mit DE-Werten im Bereich 5 bis 20 besonders bevorzugt sind. Es ist dabei unerheblich, welche Pflanze ursprünglich die Stärke zur Herstellung der Stärkehydrolysate geliefert hat. Besonders geeignet und leicht verfügbar sind jedoch Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Zubereitungen oder Halbfertigwaren, die neben der oder den erfindungsgemäß zu verwendenden Verbindungen der Formeln (I) bzw. oben definierten Mischungen noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Besonders bevorzugt sind dementsprechend erfindungsgemäße Zusammensetzungen oder Halbfertigwaren, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäße, Trägerstoffe umfassende Zubereitungen und Halbfertigwaren, die mittels Sprühtrocknung hergestellt wurden, besitzen eine mittlere Partikelgröße im Bereich von 30 bis 300 µm und eine Restfeuchte von kleiner oder gleich 5 Gew.-%.

Eine erfindungsgemäße Geschmacksstoffmischung, eine erfindungsgemäße Zubereitung oder eine erfindungsgemäße Halbfertigware (wie jeweils oben beschrieben) umfasst gemäß einem bevorzugten Aspekt der vorliegenden Erfindung - zusätzlich zu den Verbindungen der Formel (I) - eine oder mehrere Aromakomposition(en).

Eine solche Aromakomposition umfasst für die Zwecke der vorliegenden Erfindung mindestens einen flüchtigen Aromastoff (nicht gemeint sind hierbei Verbindungen der Formel (I)). Der wenigstens eine flüchtige Aromastoff ist dabei vorzugsweise eine sensorisch wirksame Komponente mit einem Dampfdruck von größer oder gleich 0,01 Pa bei 25°C, vorzugsweise einem Dampfdruck von größer oder gleich 0,025 Pa bei 25°C. Ein Großteil der flüchtigen Aromastoffe weist einen Dampfdruck von größer oder gleich 1 Pa bei 25°C auf. Diese Aromastoffe werden für die Verwendung in erfindungsgemäßen Zubereitungen, Halbfertigwaren oder Geschmacksstoffmischungen als besonders bevorzugt angesehen.

Beispiele für Aromastoffe, die Bestandteil einer oben genannten Aromakomposition sein können, finden sich z.B. in K. Bauer, D. Garbe und H. Surburg, Common Fragrance and Flavor Materials, 4th. Ed., Wiley-VCH, Weinheim 2001. Es seien beispielsweise genannt: organische Säuren (gesättigt und ungesättigt) wie z.B. Buttersäure, Essigsäure, Methylbuttersäure, Capronsäure; Alkohole (gesättigt und ungesättigt) wie z.B. Ethanol, Propylenglykol, Octenol, cis-3-Hexenol, Benzylalkohol; Sulfide und Disulfide wie z.B. Dimethylsulfid, Difurfuryldisulfid, Methylthiopropanal; Thiole wie z.B. Methylfuranthiol; Pyrazine und Pyrroline wie z.B. Methylpyrazin, Acetylpyrazin, 2-Propionylpyrrolin, 2-Acetylpyrrolin; Furanabkömmlinge wie Sotolon oder 5-Hydroxymethylfurfural.

Die Aromakompositionen können auch in Form von Reaktionsaromen (Maillard-Produkte) und/oder Extrakten bzw. ätherischen Ölen von Pflanzen oder Pflanzenteilen bzw. Fraktionen davon eingesetzt werden.

Gemäß einer weiteren bevorzugten Ausgestaltung der vorliegenden Erfindung liegt eine erfindungsgemäße Zubereitung oder Halbfertigware (wie jeweils oben beschrieben) als Wasser-in-Öl (W/O)-Emulsion vor.

Neben der oder den erfindungsgemäß zu verwendenden Verbindungen der Formel (I) umfasst eine solche Emulsion Wasser, eine Ölphase, ein oder mehrere (verzehrbare) W/O-Emulgatoren, gegebenenfalls ein oder mehrere Antioxidantien und gegebenenfalls ein oder mehrere Stoffe zur Verstärkung einer antioxidativen Wirkung. Dabei gilt hinsichtlich bevorzugter Verbindungen der Formel (I) und Mischungen davon das oben Gesagte entsprechend.

Die Ölphase einer solchen erfindungsgemäßen W/O-Emulsion besteht aus oder umfasst vorzugsweise ein fettes Öl und/oder eine Aromakomposition (vorzugsweise eine wie oben beschriebene Aromakomposition).

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Palmkernöl, Pekannussöl, Pistazienkernöl, Rapsöl, Reiskeimöl, Sesamöl, Sojaöl, Sonnenblumenöl, Walnussöl oder Weizenkeimöl, oder daraus erhältliche Fraktionen. Es können auch flüssige Neutralester basierend auf mittelkettigen Fettsäuren und Glycerin verwendet werden, wie beispielsweise Miglyole (z.B. Miglyol 810, Miglyol 812). Bevorzugt sind Sonnenblumenöl, Palmkernöl und Rapsöl. Ferner bevorzugt werden fraktionierte Kokosöle verwendet, die hauptsächlich Fettsäurereste mit 6 bis 8 C-Atomen aufweisen. Diese zeichnen sich durch ihre Geschmacksneutralität sowie durch ihre gute Oxidationsstabilität aus.

Vorzugsweise wird der verzehrbare W/O-Emulgator gewählt aus der Gruppe bestehend aus Lecithin (E 322), Mono- und Diglyceride von Speisefettsäuren (E 471), Essigsäuremonoglyceride (E 472a), Milchsäuremonoglyceride (E 472b), Citronensäuremonoglyceride (E 472c), Weinsäuremonoglyceride (E 472d), Diacetylweinsäuremonoglyceride (E 472e), Sorbitanmonostearat (E 491).

Geeignete Antioxidantien und Stoffe, welche die antioxidative Wirkung verstärken können sind insbesondere die natürlich vorkommenden Tocopherole und deren Derivate, Tocotrienole, Flavonoide, Ascorbinsäure und ihre Salze, alpha-Hydroxysäuren (z.B. Citronensäure, Milchsäure, Äpfelsäure, Weinsäure) und deren Na-, K- und Ca-Salze, aus Pflanzen isolierte Inhaltsstoffe, Extrakte bzw. Fraktionen davon, z.B. aus Tee, Grüntee, Algen, Traubenkernen, Weizenkeimen, Rosmarin, Oregano, Flavonoide, Quercetin, phenolische Benzylamine. Weiterhin sind als Antioxidantien Propylgallat, Octylgallat, Dodecylgallat, Butylhydroxyanisol (BHA), Butylhydroxytoluol (BHT), Lecithine, Mono- und Diglyceride von Speisefettsäuren verestert mit Citronensäure, Orthophosphate und Na-, K- und Ca-Salze der Monophosphorsäure und Ascorbylpalmitat geeignet.

Die erfindungsgemäßen W/O-Emulsionen eignen sich besonders zum Aufbringen auf Lebensmitteloberflächen, wobei die Lebensmittel vorzugsweise einen Wassergehalt von höchstens 10 Gew.-%, bevorzugt von höchstens 5 Gew.-% aufweisen. In einer bevorzugten Ausführungsform weist die erfindungsgemäße W/O-Emulsion bei Aufbringungstemperatur eine ausreichend niedrige Viskosität auf, so dass ein Aufbringen der W/O-Emulsion mittels Sprühen möglich ist. Bevorzugte Lebensmittel, auf deren Oberflächen eine erfindungsgemäße W/O-Emulsion aufgebracht werden kann sind beispielsweise Cracker, Chips (z.B. auf Basis von Kartoffeln, Mais, Getreide oder Brot), extrudierte Knabberartikel (Snacks, z.B. Flips) oder Laugengebäck (z.B. Salzstangen). Erfindungsgemäße W/O-Emulsionen werden regelmäßig in einer Menge von 0,5 bis 6 Gew.-% auf die Lebensmitteloberflächen aufgebracht, bezogen auf das Gesamtgewicht des Lebensmittels.

Besonders bevorzugt werden die erfindungsgemäß zu verwendenden Verbindungen der Formel (I) oder Mischungen davon bzw. erfindungsgemäße Geschmacksstoffmischungen oder pflanzliche Extrakte in Natriumglutamat-freien oder Natriumglutamat-reduzierten Zubereitungen oder Halbfertigwaren eingesetzt.

Dementsprechend sind erfindungsgemäße Natriumglutamat-freie Zubereitungen bzw. Halbfertigwaren oder Natriumglutamat-reduzierte Zubereitungen bzw. Halbfertigwaren besonders bevorzugt.

Der Begriff "Natriumglutamat-reduziert" bedeutet dabei, dass die erfindungsgemäße Zubereitung oder Halbfertigware deutlich weniger Natriumglutamat enthält, als in der üblichen Zubereitung bzw. Halbfertigware enthalten ist; der Natriumglutamatgehalt liegt dabei um 5 bis <100 Gew.-%, bevorzugt 10 bis 50 Gew.-%, besonders bevorzugt 20 bis 50 Gew.-% unter dem Natriumglutamatgehalt der üblichen Zubereitung bzw. Halbfertigware. Sofern neben einer oder mehreren Verbindungen der Formel (I) auch Natriumglutamat in einer erfindungsgemäßen Zubereitung oder Halbfertigware vorliegt, liegt das Gewichtsverhältnis der Gesamtmenge an Verbindungen der Formel (I) zu Natriumglutamat vorzugsweise im Bereich von 1 : 1 bis 1 : 200.

Besonders bevorzugt sind demnach solche Natriumglutamat-reduzierten erfindungsgemäßen Zubereitungen oder Halbfertigwaren, wobei das Gewichtsverhältnis der Gesamtmenge an Verbindungen der Formel (I) zur Gesamtmenge an Natriumglutamat, bezogen auf die Trockenmasse der Zubereitung bzw. Halbfertigware, im Bereich von 1 : 1 bis 1 : 200 liegt.

Besonders bevorzugt sind solche Natriumglutamat-reduzierten Zubereitungen (wie oben beschrieben), wobei
- die Gesamtmenge an Natriumglutamat nicht ausreicht, um in einer Vergleichszubereitung, die keine Verbindung der Formel (I) umfasst, aber ansonsten identisch zusammengesetzt ist (normale Natriumglutamat-reduzierte Zubereitung), einen (befriedigenden) Umami-Geschmackseindruck zu vermitteln,
- und die Gesamtmenge an Verbindungen der Formel (I) ausreicht, um der Zubereitung einen (befriedigenden) Umami-Geschmackseindruck sowie optional einen (befriedigenden) Kokumi- und/oder einen (befriedigenden) salzigen Geschmackseindruck zu vermitteln.

Der Ernährung oder dem Genuss dienende erfindungsgemäße Zubereitungen und erfindungsgemäße Halbfertigwaren sind vorzugsweise ausgewählt aus der Gruppe bestehend aus Backwaren (z.B. Brot, Trockenkekse, Kuchen, sonstiges Gebäck), Getränke (z.B. Gemüsesäfte, Gemüsesaftzubereitungen), Instantgetränke (z.B. Instant-Gemüsegetränke), Fleischprodukte (z.B. Schinken, Frischwurst- oder Rohwurstzubereitungen, gewürzte oder marinierte Frisch- oder Pökelfleischprodukte), gewürzte oder marinierte Fischprodukte (z.B. Surimi), Eier oder Eiprodukte (Trockenei, Eiweiß, Eigelb), Getreideprodukte (z.B. vorgegarte Fertigreis-Produkte, Reismehlprodukte, Hirse- und Sorghum-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Gemüsezubereitungen (z.B. Ketchup, Soßen, Trockengemüse, Tiefkühlgemüse, vorgegarte Gemüse, in Essig eingelegte Gemüse, Gemüsekonzentrate oder -pasten, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais-, Reis- oder Erdnussbasis), Produkte auf Fett- und Ölbasis oder Emulsionen derselben (z.B. Mayonnaise, Aufstrich, Remoulade, Dressings, Würzzubereitungen), sonstige Fertiggerichte und Suppen (z.B. Trockensuppen, Instant-Suppen, vorgegarte Suppen), Soßen (Instant-Soßen, Trockensoßen, Fertigsoßen), Gewürze oder Gewürzzubereitungen (z.B. Senfzubereitungen, Meerrettichzubereitungen), Würzmischungen sowie insbesondere Aufstreuwürzungen (englisch: Seasonings), die beispielsweise im Snackbereich Anwendung finden.

Natriumglutamat-reduzierte oder -freie der Ernährung oder dem Genuss dienende erfindungsgemäße Zubereitungen und Halbfertigwaren sind vorzugsweise ausgewählt aus der Gruppe bestehend aus Backwaren (z.B. Brot, Trockenkekse, Kuchen, sonstiges Gebäck), Gemüsesaftzubereitungen, Fleischprodukte (z.B. Schinken, Frischwurst- oder Rohwurstzubereitungen, gewürzte oder marinierte Frisch- oder Pökelfleischprodukte), gewürzte oder marinierte Fischprodukte (z.B. Surimi), Eier oder Eiprodukte (Trockenei, Eiweiß, Eigelb), Getreideprodukte (z.B. vorgegarte Fertigreis-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fischsoßen wie z.B. Anchovis-Soßen, Austernsoßen, Gemüsezubereitungen (z.B. Ketchup, Soßen, Trockengemüse, Tiefkühlgemüse, vorgegarte Gemüse, in Essig eingelegte Gemüse, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Aufstrich, Remoulade, Dressings, Würzzubereitungen), Fertiggerichte, Suppen (z.B. Trockensuppen, Instant-Suppen, vorgegarte Suppen), Brühwürfel, Soßen (Instant-Soßen, Trockensoßen, Fertigsoßen), Gewürze, Würze, Würzmittel, Würzmischungen sowie insbesondere Aufstreuwürzungen (englisch: Seasonings), die beispielsweise im Snackbereich Anwendung finden.

Die erfindungsgemäßen Zubereitungen und Halbfertigwaren können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die erfindungsgemäßen Halbfertigwaren dienen in der Regel zur Herstellung von (erfindungsgemäßen) der Ernährung oder dem Genuss dienenden gebrauchs- oder verzehrfertigen Zubereitungen.

Insbesondere können erfindungsgemäße Halbfertigwaren zur additiven Verstärkung des Umami-Geschmacks von Natriumglutamat-reduzierten Nahrungs- und Genussmitteln und auch direkt als Würzmittel für die industrielle oder nicht-industrielle Zubereitung von Nahrungs- und/oder Genussmitteln dienen.

Erfindungsgemäß besonders bevorzugt ist eine Halbfertigware (wie oben beschrieben), die bezogen auf das Gesamtgewicht der Halbfertigware eine Gesamtmenge an Natriumglutamat im Bereich von 0,00001 bis 10 Gew.-%, bevorzugt 0,0001 bis 5 Gew.-%, besonders bevorzugt von 0,001 bis 2 Gew.-%, oder kein Natriumglutamat umfasst.

Grundsätzlich enthalten erfindungsgemäß bevorzugte Halbfertigwaren vorzugsweise:
- eine Gesamtmenge von 1 ppm bis 500.000 ppm, bevorzugt 25 ppm bis 100.000 ppm, insbesondere 50 ppm bis 15.000 ppm an Verbindungen der Formel (I),
   vorzugsweise eine Gesamtmenge von 10 ppm bis 100.000 ppm, bevorzugt 25 ppm bis 5.000 ppm, insbesondere 50 ppm bis 1.200 ppm an Verbindungen der Formel (I),
- kein Natriumglutamat oder einen Anteil von 0,00001 bis 10 Gew.-%, bevorzugt 0,0001 bis 5 Gew.-%, insbesondere 0,001 Gew.-% bis 2 Gew.-% an Natriumglutamat,
- und gegebenenfalls einen Anteil von 0,0001 Gew.-% bis 90 Gew.-%, bevorzugt 0,001 Gew.-% bis 30 Gew.-% einer Aromakomposition (vorzugsweise einer Aromakomposition wie oben beschrieben),
jeweils bezogen auf das Gesamtgewicht der Halbfertigware.

Die erfindungsgemäßen Zubereitungen oder Halbfertigwaren werden vorzugsweise hergestellt, indem die Verbindungen der Formel (I) in Ethanol und gegebenenfalls demineralisiertem und/oder gereinigtem Wasser gelöst bzw. gemischt werden. Anschließend werden die Lösungen durch einen Trocknungsprozess, vorzugsweise einen Sprühtrocknungs-, Vakuumgefriertrockungs-, Umkehrosmose-, Eindampf- oder anderen Konzentrationsprozess oder eine Kombination der genannten Prozesse, in eine (zumindest nahezu) feste Form überführt. Dabei kann die Trocknung unter Zuhilfenahme von Trägerstoffen (z.B. Stärke, Stärkederivate, Maltodextrin, Kieselgel, siehe oben) oder Hilfsstoffen (z.B. Pflanzengummen, Stabilisierungsmittel) erfolgen. Vorzugsweise erfolgt die Trocknung mittels Sprühtrocknung oder Vakuumgefriertrocknung.

Bevorzugte erfindungsgemäße Zubereitungen oder Halbfertigwaren sind Würze, Würzmischungen, Würzmittel, Brühwürfel, Instant-Suppen, Instant-Soßen, vegetarische Fertiggerichte, fleischhaltige Fertiggerichte, Fischsoßen wie z.B. Anchovis-Soßen, Austernsoßen und Sojasoßen.

Gemäß einer weiteren bevorzugten Ausführungsform werden zur Herstellung erfindungsgemäßer Zubereitungen oder Halbfertigwaren Verbindungen der Formel (I) sowie gegebenenfalls andere Bestandteile zunächst in Emulsionen, in Liposomen (z.B. ausgehend von Phosphatidylcholin) in Microsphären, in Nanosphären oder auch in Kapseln, Granulate oder Extrudate aus einer für Lebens- und Genussmittel geeigneten Matrix (z.B. aus Stärke, Stärkederivaten, Cellulose oder Cellulosederivaten wie Hydroxypropylcellulose, anderen Polysacchariden wie Alginat, natürlichen Fetten, natürlichen Wachsen wie Bienenwachs oder Carnaubawachs oder aus Proteinen wie Gelatine) eingearbeitet.

In einem weiteren bevorzugten Herstellungsverfahren werden Verbindungen der Formel (I) mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Besonders bevorzugt sind erfindungsgemäße Zubereitungen oder Halbfertigwaren, bei denen die Matrix so gewählt ist, dass die Verbindungen der Formel (I) verzögert von der Matrix freigegeben werden, so dass man eine langanhaltende Wirkung erhält. Als Matrix können hier z.B. natürliche Fette, natürliche Wachse (z.B. Bienenwachs, Carnaubawachs) oder auch natürliche Ballaststoffe (Weizenfasern, Apfelfasern, Haferfasern, Orangenfasern) verwendet werden.

Weitere Bestandteile einer erfindungsgemäßen, der Ernährung oder dem Genuss dienenden verzehrfertigen Zubereitung oder einer erfindungsgemäßen Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (s. hierzu auch oben; z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße Zubereitungen oder Halbfertigwaren zudem eine Aromakomposition (wie oben beschrieben), um den Geschmack und/oder den Geruch abzurunden und zu verfeinern.

Geeignete Aromakompositionen enthalten vorzugsweise synthetische, natürliche oder naturidentische Aroma-, Riech- und Geschmacksstoffe, Reaktionsaromen, Raucharomen oder andere aromagebende Zubereitungen (z.B. Protein[teil]hydrolysate, Grillaromen, Pflanzenextrakte, Gewürze, Gewürzzubereitungen, Gemüse und/oder Gemüsezubereitungen) sowie gegebenenfalls geeignete Hilfs- und Trägerstoffe. Insbesondere sind hier solche Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

Besonders bevorzugt umfasst eine erfindungsgemäße Geschmacksstoffmischung, eine erfindungsgemäße Zubereitung oder eine erfindungsgemäße Halbfertigware zudem eine oder mehrere nicht der Formel (I) entsprechende Substanzen zum Maskieren oder Vermindern eines unangenehmen, insbesondere eines bitteren, metallischen, kalkigen, sauren und/oder adstringierenden Geschmackseindrucks, und/oder eine oder mehrere nicht der Formel (I) entsprechende Substanzen zum Vermitteln oder Verstärken eines angenehmen Geschmackseindrucks, insbesondere eines süßen oder salzigen Geschmackseindrucks, eines Kokumi-Geschmackseindrucks oder eines Umami-Geschmackseindrucks.

Gemäß einem weiteren Aspekt der vorliegenden Erfindung umfasst eine erfindungsgemäße Geschmacksstoffmischung, eine erfindungsgemäße Zubereitung oder eine erfindungsgemäße Halbfertigware (zudem) eine oder mehrere nicht der Formel (I) entsprechende Geschmacksstoffe, die einen nicht-scharfen und/oder nicht-wärmenden trigeminalen Reiz, insbesondere einen Reiz ausgewählt aus der Gruppe bestehend aus tingling, kribbelnd, stechend, kühlend und adstringierend, vermitteln, wobei der nicht der Formel (I) entsprechende Geschmacksstoff bzw. einer, mehrere oder sämtliche der nicht der Formel (I) entsprechenden Geschmacksstoffe vorzugsweise ausgewählt ist bzw. sind aus der Gruppe bestehend aus Hesperetin, Phloretin, cis-Pellitorin und trans-Pellitorin. Besonders bevorzugt ist eine cis- und/oder trans-Pellitorin enthaltende erfindungsgemäße Geschmacksstoffmischung, Zubereitung bzw. Halbfertigware, wobei die Gesamtmenge an Pellitorin bezogen auf das Gesamtgewicht der Geschmacksstoffmischung, der Zubereitung bzw. der Halbfertigware vorzugsweise im Bereich von 0,5 bis 500 ppm, bevorzugt im Bereich von 5 bis 100 ppm, liegt.

So kann beispielsweise bei der Kombination der erfindungsgemäß zu verwendenden Verbindungen der Formel (I) mit Hesperetin und/oder Phloretin einerseits und cis- und/oder trans-Pellitorin (siehe WO 2004/000787 bzw. WO 2004/043906) andererseits ein weiter verbessertes und von Konsumenten bevorzugtes Geschmacksprofil erreicht werden.

Modulierende Aroma- und/oder Geschmackstoffe werden vorzugsweise ausgewählt aus der Gruppe bestehend aus Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoesäureamiden, wie zum Beispiel 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N-*(4-hydroxy-3-methoxybenzyl)-amid, 2,4-Dihydroxybenzoesäure-*N*-(4-hydroxy-3-methoxybenzyl)amid-Mono-natriumsalz, 2,4-Dihydroxybenzoesäure-*N*-2-(4-hydroxy-3-methoxyphenyl)-ethyl-amid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl) ethanon) (insbesondere solche wie beschrieben in WO 2006/106023 und DE 10 2009 002 268.6, die bezüglich der darin offenbarten entsprechenden Verbindungen auf dem Wege der Verweisung Bestandteil dieser Anmeldung wird); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); γ-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird) und Divanillinen (insbesondere Divanillin wie beschrieben in WO 2004/078302, die bezüglich der darin offenbarten entsprechenden Verbindungen auf dem Wege der Verweisung Bestandteil dieser Anmeldung wird); Bicyclo[4.1.0]heptan-7-carbonsäureamide, insbesondere solche wie beschrieben in PCT/EP2007/061171 sowie den darauf basierenden Dokumenten (Symrise), die bezüglich der darin offenbarten entsprechenden Verbindungen auf dem Wege der Verweisung Bestandteil dieser Anmeldung wird; Cyclopropancarbonsäure(3-methyl-cyclohexyl)amide, insbesondere solche wie beschrieben in US-Provisional 60/916,589 vom 08.05.2007 sowie den darauf basierenden Patent-Dokumenten (Symrise), die bezüglich der darin offenbarten entsprechenden Verbindungen auf dem Wege der Verweisung Bestandteil dieser Anmeldung wird; Aromatische Neo-Menthylamide, insbesondere solche wie beschrieben in US-Provisional Application 60/984,023 vom 31.10.2007 sowie den darauf basierenden Dokumenten (Symrise), die bezüglich der darin offenbarten entsprechenden Verbindungen auf dem Wege der Verweisung Bestandteil dieser Anmeldung wird; Geranylaminederivate der Oxalsäure, insbesondere solche wie beschrieben in EP 2 168 442 sowie den darauf basierenden Dokumenten (Symrise), die bezüglich der darin offenbarten entsprechenden Verbindungen auf dem Wege der Verweisung Bestandteil dieser Anmeldung wird; Neomenthylderivate wie beschrieben in US 2009 0311401-A1 sowie den darauf basierenden Patent-Dokumenten (Symrise), die bezüglich der darin offenbarten entsprechenden Verbindungen auf dem Wege der Verweisung Bestandteil dieser Anmeldung wird.

Im Zusammenhang mit der vorliegenden Erfindung wird auch ein Verfahren zum Vermitteln, Modifizieren und/oder Verstärken eines Umami-, Kokumi- und/oder salzigen Geschmacks einer der Ernährung oder dem Genuss dienenden gebrauchs- oder verzehrfertigen Zubereitung oder Halbfertigware beschrieben. Ein solches Verfahren umfasst den Schritt
- Vermischen einer Umami-geschmacklich wirksamen Menge (für bevorzugte Mengen s. oben), einer oder mehrerer erfindungsgemäß zu verwendender Verbindungen der Formel (I) oder einer erfindungsgemäßen Geschmacksstoffmischung (wie oben beschrieben) oder eines erfindungsgemäßen pflanzlichen Extrakts (wie oben beschrieben) mit einem oder mehreren weiteren Bestandteilen der Zubereitung bzw. Halbfertigware, oder
- Applizieren einer Umami-geschmacklich wirksamen Menge einer oder mehrerer Verbindungen der Formel (I) oder einer erfindungsgemäßen Geschmacksstoffmischung oder eines erfindungsgemäßen pflanzlichen Extrakts (wie jeweils oben beschrieben) auf einen oder mehrere weitere Bestandteile der Zubereitung bzw. Halbfertigware, oder
- Einbetten einer geschmacklich wirksamen Menge einer oder mehrerer Verbindungen der Formel (I) oder einer erfindungsgemäßen Geschmacksstoffmischung oder eines erfindungsgemäßen pflanzlichen Extrakts (wie jeweils oben beschrieben) in ein Hüll- oder Matrixmaterial.

Dabei gilt hinsichtlich bevorzugter Verbindungen der Formel (I) bzw. Bestandteile und Mengen das oben Gesagte entsprechend.

Weitere Aspekte der vorliegenden Erfindung ergeben sich aus den nachfolgenden Beispielen und den beigefügten Ansprüchen.

### Beispiele:

Die Beispiele dienen zur Verdeutlichung der Erfindung, ohne diese damit einzuschränken. Sofern nicht anders angegeben, beziehen sich alle Angaben auf das Gewicht.

### Beispiel 1: Einfache Profilierung beispielhafter Zimtamide (Verbindungen (1), (3) und (9))

Die Verbindungen wurden in Wasser in der angegebenen Konzentration gelöst und durch eine Expertengruppe durch Verkostung in freier Diskussion bewertet.

| **Struktur** | **Name (Verbindungsnummer)** | **Geschmacksprofil (Konzentration in mg/kg)** |
|---|---|---|
| 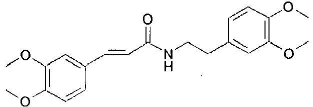 | Rubemamin (1) | Umami (10 mg/kg) |
| 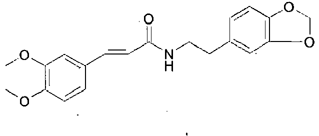 | Rubescenamin (3) | Umami, sonst neutral (10 mg/kg) |
| 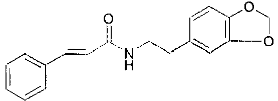 | Zanthomamin (9) | etwas verzögert einsetzende Umami-Wirkung (50 mg/kg) |

### Beispiel 2: Profilierung beispielhafter Zimtamide (Verbindungen (1) und (3)) in einer komplexen Base

Ein amerikanischer Rindfleischextrakt als Base, eine entsprechende mit Natriumglutamat versetzte Base und eine entsprechende mit dem Zimtamid versetzte Base wurden jeweils im Einzelnen geschulten 15-25 Panelisten blind zur Profilierung dargereicht. Anhand der vorher in Diskussionen festgelegten Deskriptoren (Mundfülle, salzig, metallisch, fleischig, bitter, mundwässernd, bratig-röstig, süß, sauer, haftfest) wurde deren Stärke anhand einer Skala von 0 (nicht_wahrnehmbar) bis 9 (sehr stark) eingeschätzt. Die Einzelergebnisse der Panelisten wurden gemittelt.

In dem als Figur 1 beigefügten Netzdiagramm wird das Profil eines amerikanischen Rindfleischextrakts als Base (i) mit einer solchen Base mit einem Zusatz von 10 ppm (iii) bzw. 50 ppm Rubemamin (Verbindung (3)) (iv) und mit einer Probe (ii) einer solchen Base mit einem Zusatz von 0,05 Gew.-% MSG (Natriumglutamat) verglichen.

In dem als Figur 2 beigefügten Netzdiagramm wird das Profil eines amerikanischen Rindfleischextrakts als Base (i) mit einer solchen Base mit einem Zusatz von 5 ppm (iii) bzw. 10 ppm Rubescenamin (Verbindung (3)) (iv) und mit einer Probe (ii) einer solchen Base mit einem Zusatz von 0,05 Gew.-% MSG (Natriumglutamat) verglichen.

### Beispiel 3: Profilierung beispielhafter Zimtamide (Verbindungen (1), (3) und (9)) in einer komplexen Base zusammen mit Mononatriumglutamat (Verstärkungseffekt)

Ein amerikanischer Rindfleischextrakt als Base, eine entsprechende mit Natriumglutamat versetzte Soße, eine entsprechende mit dem Zimtamid versetzte Soße und eine entsprechende mit Natriumglutamat versetzte Base wurde, jeweils im Einzelnen geschulten 15-25 Panelisten blind zur Profilierung dargereicht. Anhand der vorher in Diskussionen festgelegten Deskriptoren (Mundfülle, salzig, metallisch, fleischig, bitter, mundwässernd, bratig-röstig, süß, sauer, haftfest) wurde deren Stärke anhand einer Skala von 0 (nicht wahrnehmbar) bis 9 (sehr stark) eingeschätzt. Die Einzelergebnisse der Panelisten wurden gemittelt.

Die Verstärkung des Natriumglutamat-Effekts wird durch das als Figur 3 beigefügte Netzdiagramm belegt. Hier wird (i) der Rindfleischextrakt als Base mit (ii) einer solchen Base unter Zusatz von 0,05 % Natriumglutamat, (iii) einer solchen Base unter Zusatz von 0,0025 % Natriumglutamat und (iv) einer solchen Base unter Zusatz von 0,0025 % Natriumglutamat und 20 ppm Rubemamin (1) verglichen. Während die Probe (iii) insgesamt bei den abgefragten Parametern nur wenig stärker als die Probe (i) ohne Zusatz wahrgenommen wurde, sind die stark Glutamat-haltige Probe (ii) und die Probe mit geringer Konzentration Glutamat und Rubemamin (iv) bis auf eine etwas geringere Süße nahezu nicht unterscheidbar.

Insbesondere Rubescenamin (Verbindung (3)) ist vorteilhafterweise auch in besonders geringer Konzentration geeignet, den Natriumglutamat-Effekt zu verstärken, wie durch das als Figur 4 beigefügte Netzdiagramm belegt wird. Hier wird wiederum (i) ein Rindfleischextrakt als Base mit (ii) einer solchen Base unter Zusatz von 0,05 % Natriumglutamat, (iii) einer solchen Base unter Zusatz von 0,0025 % Natriumglutamat und (iv) einer solchen Base unter Zusatz von 0,0025 % Natriumglutamat und 5 ppm Rubescenamin (3) verglichen. Auch hier wurde die Probe mit geringer Konzentration Glutamat und Rubescenamin (iv) in nahezu allen Parametern stärker als Probe (iii) und insbesondere für den Parameter "salzig" ähnlich der Probe (ii) bewertet.

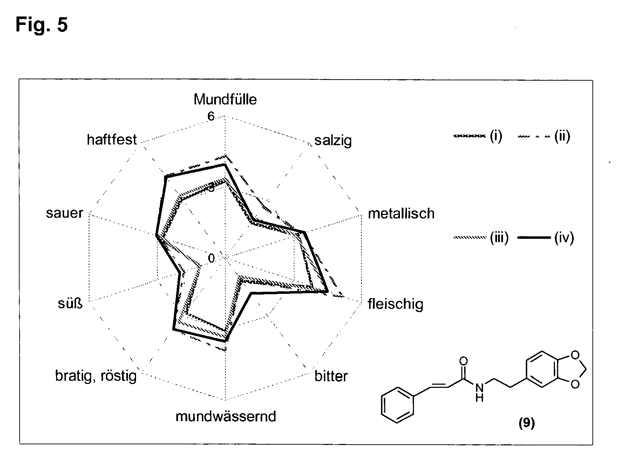

Insbesondere Zanthomamin (Verbindung (9)) ist ebenfalls besonders geeignet, den Natriumglutamat-Effekt zu verstärken, wie durch das als Figur 5 beigefügte Netzdiagramm belegt wird. Hier wird wiederum (i) ein Rindfleischextrakt als Base mit (ii) einer solchen Base unter Zusatz von 0,05 % Natriumglutamat, (iii) einer solchen Base unter Zusatz von 0,0025 % Natriumglutamat und (iv) einer solchen Base unter Zusatz von 0,0025 % Natriumglutamat und mit einem Zusatz von 50 ppm Zanthomamin (9) verglichen. Auch hier wurde die Probe mit geringer Konzentration Glutamat und Zanthomamin (iv) in nahezu allen Parametern stärker als Probe (iii) bewertet.

### Anwendungsbeispiele

Die Anwendungsbeispiele dienen zur Verdeutlichung der Erfindung, ohne diese damit einzuschränken. Sofern nicht anders angegeben, beziehen sich alle Angaben auf das Gewicht.

### Anwendungsbeispiel 1: Sprühgetrocknete Zusammensetzungen

**1.1**

| **Bestandteil** | **Anteil** |
|---|---|
| Rubemamin (1) | 10 g |
| Maltodextrin | 90 g |

**1.2**

| **Bestandteil** | **Anteil** |
|---|---|
| Rubescenamin (3) | 10 g |
| Maltodextrin | 90 g |

Die Bestandteile werden in einer Mischung aus Ethanol und demineralisiertem Wasser gelöst und anschließend sprühgetrocknet. Die Zusammensetzungen werden in nachfolgend beschriebenen Anwendungsbeispielen eingesetzt.

### Anwendungsbeispiel 2: Aromakomposition, nicht erfindungsgemäß

| **Inhaltsstoff** | **Anteil** |
|---|---|
| 10 Gew.-% trans-Pellitorin in 1,2-Propylenglycol/Diethylmalonat | 0,25 g |
| Hesperetin | 2,50 g |
| Phloretin | 1,50 g |
| Propylenglycol | 95,75 g |

Die Aromakomposition wird in nachfolgend beschriebenen Anwendungsbeispielen eingesetzt.

### Anwendungsbeispiel 3: Würzmittel

| **Teil** | **Bestandteil** | **Anteil** |
|---|---|---|
| A | Rubemamin (1) | 0,2g |
| | Natriumchlorid | 15g |
| B | Senfsamenmehl | 5g |
| | Senfaroma | 0,1g |

Teil A wird eingewogen. Es werden 290 ml Wasser vorgelegt und unter Rühren Teil A zugegeben und gelöst. Die Lösung wird mit Wasser auf 1,84 kg verdünnt (pH 6,0) und anschließend gefriergetrocknet (Eutektischer Punkt: -15°C; Arbeitsvakuum: 0,52 mbar; Stellflächentemperatur: -5°C bis +25°C). Das Produkt wird mit Senfsamenmehl und dem Senfaroma aus Teil B gemischt und zu einem Würzmittel konfektioniert.

### Anwendungsbeispiel 4: Reaktionsaroma

| **Inhaltsstoff** | **Einsatz [g]** | | | |
|---|---|---|---|---|
| | Ausführung | Ausführung | Ausführung | Ausführung |
| | A | B | C | D |
| L-Alanin | 41,0 | 41,0 | 41,0 | 41,0 |
| L-Asparaginsäure | 123,0 | 123,0 | 123,0 | 123,0 |
| Bernsteinsäure | 4,7 | 4,7 | 4,7 | 4,7 |
| Calciumchlorid Dihydrat | 7,0 | 7,0 | 7,0 | 7,0 |
| L-Cystein•Cl Monohydrat | 11,0 | 11,0 | 11,0 | 11,0 |
| Dikaliumphosphat | 6,0 | 6,0 | 6,0 | 6,0 |
| Fructose gemahlen | 1,0 | 1,0 | 1,0 | 1,0 |
| L-Isoleucin | 1,6 | 1,6 | 1,6 | 1,6 |
| Kaliumchlorid | 228,0 | 228,0 | 228,0 | 228,0 |
| L-Leucin | 1,6 | 1,6 | 1,6 | 1,6 |
| L-Lysin•HCl | 3,6 | 3,6 | 3,6 | 3,6 |

| **Inhaltsstoff** | **Einsatz [g]** | | | |
|---|---|---|---|---|
| Magnesiumchlorid Hexahydrat | 19,0 | 19,0 | 19,0 | 19,0 |
| Maltodextrin | 49,0 | 49,0 | 49,0 | 49,0 |
| L-Phenylalanin | 2,0 | 2,0 | 2,0 | 2,0 |
| L-Prolin | 74,0 | 74,0 | 74,0 | 74,0 |
| L-Serin | 6,5 | 6,5 | 6,5 | 6,5 |
| L-Threonin | 3,0 | 3,0 | 3,0 | 3,0 |
| L-Valin | 9,0 | 9,0 | 9,0 | 9,0 |
| Wasser | 384,0 | 389,0 | 379,0 | 379,0 |
| Rubemamin (1) | 25,0 | 0 | 20 | 0 |
| Rubescenamin (3) | 0 | 20 | 10 | 0 |
| Zanthomamin (9) | 0 | 0 | 0 | 30 |

Alle Komponenten werden bei 40°C gemischt und anschließend bei 85°C für 10 Minuten erhitzt (Rückflussreaktion). Nach dem Abkühlen auf 40°C wird mit Kalilauge auf pH 5 eingestellt. Diese "Umami"-Reaktionsaromen können anstelle der reinen Verbindungen der Formel (I) in die Bouillon - Zubereitungen C bzw. D des Anwendungsbeispiels 9 eingearbeitet werden, wobei es bevorzugt ist in Zubereitung C 5 g und in Zubereitung D 13 g des "Umami"-Reaktionsaromas zu verwenden.

### Anwendungsbeispiel 5: Instantsuppe, Typ Lauch-Creme

A = Vergleichszubereitung
B, C, D = erfindungsgemäße Zubereitungen (Natriumglutamatfrei)
E = erfindungsgemäße Zubereitungen (Salzreduziert und Natriumglutamatfrei)

| **Bestandteil** | **A** | **B** | **C** | **D** | **E** |
|---|---|---|---|---|---|
| Kartoffelstärke | 20,0 g | 20,0 g | 20,0 g | 20,0 g | 20,0 g |
| Fettpulver | 25,0 g | 25,0 g | 25,0 g | 25,0 g | 25,0 g |
| Lactose | 20,0 g | 20,0 g | 20,0 g | 20,0 g | 20,0 g |
| Maltodextrin | 11, 730 g | 14,714 g | 14,710 g | 14,680 g | 15,705 g |
| Kochsalz | 8,0 g | 8,0 g | 8,0 g | 8,0 g | 7,0 g |
| Natriumglutamat | 3,0 g | - | - | - | - |
| Spinatpulver | 2,0 g | 2,0 g | 2,0 g | 2,0 g | 2,0 g |
| Grünes Lauchpulver | 2,0 g | 2,0 g | 2,0 g | 2,0 g | 2,0 g |
| Citronensäure, als Pulver | 0,3 g | 0,3 g | 0,3 g | 0,3 g | 0,3 g |
| Gehärtetes Pflanzenfett | 3,0 g | 3,0 g | 3,0 g | 3,0 g | 3,0 g |
| Gefriergetrockneter Lauch | 1,0 g | 1,0 g | 1,0 g | 1,0 g | 1,0 g |
| Huhnaroma | 1,0 g | 1,0 g | 1,0 g | 1,0 g | 1,0 g |
| Würzmischung Typ "grüner Lauch", Pulver | 2,0 g | 2,0 g | 2,0 g | 2,0 g | 2,0 g |
| Würzmischung, Typ "gekochte Zwiebel" | 0,6 g | 0,6 g | 0,6 g | 0,6 g | 0,6 g |
| Hefe-Würzmischung, Typ "Gemüsebrühe", Pulver | 0,3 g | 0,3 g | 0,3 g | 0,3 g | 0,3 g |
| Curcuma-Extrakt | 0,07 g | 0,07 g | 0,07 g | 0,07 g | 0,07 g |
| Rubemamin (1) | - | 0,0025 g | - | 0,050 g | 0,015 g |
| Rubescenamin (3) | - | - | 0,0025 g | - | 0,010 g |

5 g der jeweiligen Pulvermischung werden mit je 100 ml heißem Wasser aufgegossen, um eine verzehrfertige Suppe zu erhalten.

### Anwendungsbeispiel 6: Instantsuppe, Typ Hühnersuppe mit Nudeln

A = Vergleichszubereitung
B, C, D = erfindungsgemäße Zubereitungen (Natriumglutamat-frei)
E = erfindungsgemäße Zubereitungen (Salz-reduziert und Natriumglutamat-frei)

| **Bestandteil** | **A** | **B** | **C** | **D** | **E** |
|---|---|---|---|---|---|
| Stärke | 16,0 g | 16,0 g | 16,0 g | 16,0 g | 16,0 g |
| Kochsalz | 7 g | 7g | 7g | 7 g | 5 g |
| Saccharose, raffiniert | 3,2 g | 3,2 g | 3,2 g | 3,2 g | 3,2 g |
| Natriumglutamat | 3,2 g | - | - | - | - |
| Natriuminosinat / Natriumguanylat im Verhältnis 1:1 | 0,8 g | 0,8 g | 0,8 g | 0,8 g | 0,8 g |
| Säurehydrolysiertes Pflanzenprotein | 8,0 g | 8,0 g | 8,0 g | 8,0 g | 8,0 g |
| Fettpulver | 2,0 g | 2,0 g | 2,0 g | 2,0 g | 2,0 g |
| Gemüsefett, sprühgetrocknet | 1,0 g | 1,0 g | 1,0 g | 1,0 g | 1,0 g |
| Gefriergetrocknetes Hühnerfleisch, in Stückchen | 2,0 g | 2,0 g | 2,0 g | 2,0 g | 2,0 g |
| Suppen-Nudeln | 32,0 g | 32,0 g | 32,0 g | 32,0 g | 32,0 g |
| Maltodextrin | 12,160 g | 15,339 g | 14,135 g | 14,110 g | 15,144 g |
| Chinesisches Gemüse, gefriergetrocknet | 4,6 g | 4,6 g | 4,6 g | 4,6 g | 4,6 g |
| Huhnaroma | 8,0 g | 8,0 g | 8,0 g | 8,0 g | 8,0 g |
| Lebensmittelfarbstoff Riboflavin | 0.04 g | 0.04 g | 0.04 g | 0.04 g | 0.04 g |
| Rubemamin (1) | - | 0,0025 g | 0,025 g | 0,05 g | 0,015 g |
| Aromenkomposition nach Anwendungsbeispiel 2 | - | - | 1,2 g | 1,2 g | 1,2 g |

4,6 g der jeweiligen Pulvermischung werden 10 Minuten in je 100 ml Wasser gekocht, um eine verzehrfertige Suppe zu erhalten.

### Anwendungsbeispiel 7: Würzmischung, Typ "Pfeffer":

A = Vergleichszubereitung
B, C, D = erfindungsgemäße Zubereitungen (Natriumglutamat-frei)
E = erfindungsgemäße Zubereitungen (Salz-reduziert und Natriumglutamat-frei)

| **Bestandteil** | **A** | **B** | **C** | **D** | **E** |
|---|---|---|---|---|---|
| Milchprotein | 0,8 g | 0,8 g | 0,8 g | 0,8 g | 0,8 g |
| Johannisbrotkernmehl | 2,0 g | 2,0 g | 2,0 g | 2,0 g | 2,0 g |
| Maisstärke | 22,0 g | 27,995 g | 27,965 g | 27,340 g | 29,900 g |
| Kochsalz | 14,0 g | 14,0 g | 14,0 g | 14,0 g | 12,0 g |
| Paprikapulver | 13,0 g | 13,0 g | 13,0 g | 13,0 g | 13,0 g |
| Tomatenpulver | 13,0 g | 13,0 g | 13,0 g | 13,0 g | 13,0 g |
| Saccharose | 4,0 g | 4,0 g | 4,0 g | 4,0 g | 4,0 g |
| Knoblauchpulver | 0,5 g | 0,5 g | 0,5 g | 0,5 g | 0,5 g |
| Gehärtetes Pflanzenfett | 8,0 g | 8,0 g | 8,0 g | 8,0 g | 8,0 g |
| Fettpulver | 11,0 g | 11,0 g | 11,0 g | 11,0 g | 11,0 g |
| Natriumglutamat | 6,0 g | - | - | - | - |
| Lebensmittelfarbstoff Rote Bete und Paprika | 2,0 g | 2,0 g | 2,0 g | 2,0 g | 2,0 g |
| Aroma Typ "Pfeffer" | 2,0 g | 2,0 g | 2,0 g | 2,0 g | 2,0 g |
| Aroma Typ "Pizza" | 1,2 g | 1,2 g | 1,2 g | 1,2 g | 1,2 g |
| Aroma Typ "Tomate" | 0,4 g | 0,4 g | 0,4 g | 0,4 g | 0,4 g |
| Extrakt aus schwarzem Pfeffer | 0,1 g | 0,1 g | 0,1 g | 0,1 g | 0,1 g |
| Rubemamin (1) | - | 0,005 g | - | 0,060 g | 0,10 g |
| Rubescenamin (3) | - | - | 0,035 g | 0,060 g | - |

Jeweils 100 g Nackensteak vom Schwein werden mit jeweils 1,7 g der Zubereitungen A, B, C und D gleichmäßig bestreut und gebraten.

### Anwendungsbeispiel 8: Tomatenketchup

A = Vergleichszubereitung
B = Vergleichszubereitung (Salz- und Zucker-reduziert, Natriumglutamat-frei)
C = erfindungsgemäße Zubereitung (Natriumglutamatfrei, Zucker-reduziert)
D = erfindungsgemäße Zubereitung (Salz- und Zucker-reduziert, Natriumglutamatfrei)

| **Bestandteil** | **A** | **B** | **C** | **D** |
|---|---|---|---|---|
| Natriumglutamat | 0,40 g | - | - | - |
| Kochsalz | 2 g | 1 g | 2 g | 1 g |
| Stärke, Farinex WM 55 | 1 g | 1 g | 1 g | 1 g |
| Sucrose | 12 g | 9,2 g | 9,2 g | 9,2 g |
| Tomaten-Konzentrat 2-fach | 40 g | 40 g | 40 g | 40 g |
| Glucosesirup 80 Brix | 18 g | 18 g | 18 g | 18 g |
| Branntweinessig 10% | 7g | 7 g | 7 g | 7 g |
| Wasser | 19,60 g | 23,80 g | 22,30 g | 23.25 g |
| Aromenkomposition nach Anwendungs-beispiel 2.1 | - | - | 0,4 g | 0.4 g |
| 5 %ige Lösung Rubemamin (1) in Propylenglycol | - | - | 0,10 g | - |
| 5 %ige Lösung Rubescenamin (3) in Propylenglycol | - | - | - | 0,15 g |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 9: Bouillon

A = Vergleichszubereitung
B = Vergleichszubereitung (Natriumglutamat-reduziert)
C = erfindungsgemäße Zubereitung
D = erfindungsgemäße Zubereitung (Natriumglutamat-frei)
E = erfindungsgemäße Zubereitung (Salz-reduziert und Natriumglutamat-frei)

| **Bestandteil** | **A** | **B** | **C** | **D** | **E** |
|---|---|---|---|---|---|
| Fettpulver | 8,77 g | 8,77 g | 8,77 g | 8,77 g | 8,77 g |
| Natriumglutamat | 8,77 g | 5g | 5g | - | - |
| Hefeextrakt Pulver | 12,28 g | 12,28 g | 12,28 g | 12,28 g | 12,28 g |
| Kochsalz | 29,83 g | 29,83 g | 29,83 g | 29,83 g | 26,83 g |
| Maltodextrin | 37,28 g | 41,050 g | 41,040 g | 45,090 g | 48,950 g |
| Natürlicher Gemüseextrakt | 3,07 g | 3,07 g | 3,07 g | 3,07 g | 3,07 g |
| Rubemamin (1) | - | - | 0,010 g | 0,030 g | - |
| Rubescenamin (3) | - | - | - | 0,030 g | 0,100 g |

15 g der jeweiligen Pulvermischung werden mit je 1000 ml heißem Wasser aufgegossen.

### Anwendungsbeispiel 10: Würzmischung für Kartoffelchips

A = Vergleichszubereitung
B = Vergleichszubereitung (Natriumglutamat-reduziert)
C = erfindungsgemäße Zubereitung (Natriumglutamat-reduziert)
D = erfindungsgemäße Zubereitung (Natriumglutamat-frei)
E = erfindungsgemäße Zubereitung (Natriumglutamat- und Salz-reduziert)

| **Bestandteil** | **A** | **B** | **C** | **D** | **E** |
|---|---|---|---|---|---|
| Natriumglutamat | 3,50 g | 2,00 g | 2,00 g | - | 1,00 g |
| Käsepulver | 10,00 g | 10,00 g | 10,00 g | 10,00 g | 10,00 g |
| Knoblauchpulver | 2,00 g | 2,00 g | 2,00 g | 2,00 g | 2,00 g |
| Molkenpulver | 38,86 g | 40,36 g | 40,06 g | 41,91 g | 44,76 g |
| Würzextraktöl | 0,20 g | 0,20 g | 0,20 g | 0,20 g | 0,20 g |
| Paprikapulver | 9,80 g | 9,80 g | 9,80 g | 9,80 g | 9,80 g |
| Kochsalz | 21,00 g | 21,00 g | 21,00 g | 21,00 g | 17,00 g |
| Tomatenpulver | 9,00 g | 9,00 g | 9,00 g | 9,00 g | 9,00 g |
| Trockenaroma | 2,50 g | 2,50 g | 2,50 g | 2,50 g | 2,50 g |
| Siliciumdioxid | 0,02 g | 0,02 g | 0,02 g | 0,02 g | 0,02 g |
| Pflanzenöl | 0,02 g | 0,02 g | 0,02 g | 0,02 g | 0,02 g |
| Zwiebelpulver | 3,00 g | 3,00 g | 3,00 g | 3,00 g | 3,00 g |
| Sahne Aromakonzentrat | 0,03 g | 0,03 g | 0,03 g | 0,03 g | 0,03 g |
| Käse Aroma | 0,03 g | 0,03 g | 0,03 g | 0,03 g | 0,03 g |
| Tomaten Aromakonzentrat | 0,04 g | 0,04 g | 0,04 g | 0,04 g | 0,04 g |
| Sprühgetrocknete Zusammen-setzung gemäß Beispiel 1.1 | - | - | 0,30 g | - | 0,30 g |
| Sprühgetrocknete Zusammen-setzung gemäß Beispiel 1.2 | - | - | - | 0,45 g | 0,30 g |

6 g der Würzmischung werden auf 94 g Kartoffelchips aufgezogen.

### Anwendungsbeispiel 11: Weiße Soße

A = Vergleichszubereitung
B = Vergleichszubereitung (Natriumglutamat-reduziert)
C = erfindungsgemäße Zubereitung (Natriumglutamat-reduziert)
D = erfindungsgemäße Zubereitung (Natriumglutamat-frei)
E = erfindungsgemäße Zubereitung (Natriumglutamat- und Salz-reduziert)

| **Bestandteil** | **A** | **B** | **C** | **D** | **E** |
|---|---|---|---|---|---|
| Maltodextrin | 25,98 g | 27,18 g | 27,08 g | 27,58 g | 28,43 g |
| Kochsalz | 7,50 g | 7,50 g | 7,50 g | 7,50 g | 6,00 g |
| Natriumglutamat | 2,00 g | 0,80 g | 0,80 g | - | 0,80 g |
| Pflanzenfett | 5,00 g | 5,00 g | 5,00 g | 5,00 g | 5,00 g |
| Pfeffer, weiß | 0,02 g | 0,02 g | 0,02 g | 0,02 g | 0,02 g |
| Zwiebelpulver | 1,50 g | 1,50 g | 1,50 g | 1,50 g | 1,50 g |
| vorverkleisterte Maisstärke | 30,00 g | 30,00 g | 30,00 g | 30,00 g | 30,00 g |
| Fettpulver | 28,00 g | 28,00 g | 28,00 g | 28,00 g | 28,00 g |
| Sprühgetrocknete Zusammensetzung gemäß Beispiel 1.1 | - | - | 0,10 g | - | 0,20 g |
| Sprühgetrocknete Zusammensetzung gemäß Beispiel 1.2 | - | - | - | 0,40 g | 0,05 g |

90 g der Soßenmischung werden mit 1000 ml heißem Wasser aufgegossen und mit dem Schneebesen kräftig verrührt.

### Anwendungsbeispiel 12: Braune Soße

A = Vergleichszubereitung
B = Vergleichszubereitung (Natriumglutamat-reduziert)
C = erfindungsgemäße Zubereitung (Natriumglutamat-reduziert)
D = erfindungsgemäße Zubereitung (Natriumglutamat-frei)
E = erfindungsgemäße Zubereitung (Natriumglutamat- und Salz-reduziert)

| **Bestandteil** | **A** | **B** | **C** | **D** | **E** |
|---|---|---|---|---|---|
| Stärke | 40,00 g | 40,00 g | 40,00 g | 40,00 g | 40,00 g |
| Maltodextrin | 33,10 g | 33,80 g | 33,66 g | 34,70 g | 35,07 g |
| Kochsalz | 6,00 g | 6,00 g | 6,00 g | 6,00 g | 4,50 g |
| Zuckerkulör, sprühgetrocknet | 5,00 g | 5,00g | 5,00 g | 5,00 g | 5,00 g |
| Hefeextraktpulver | 3,00 g | 3,00 g | 3,00 g | 3,00 g | 3,00 g |
| Natriumglutamat | 2,00 g | 1,30 g | 1,30 g | - | 1,30g |
| Zucker | 0,50 g | 0,50 g | 0,50 g | 0,50 g | 0,50 g |
| Fettpulver | 5,00 g | 5,00 g | 5,00 g | 5,00 g | 5,00 g |
| Tomatenpulver | 3,00 g | 3,00g | 3,00 g | 3,00 g | 3,00 g |
| Natürlicher Gemüseextrakt | 1,00 g | 1,00 g | 1,00 g | 1,00 g | 1,00 g |
| Zwiebelextrakt | 0,30 g | 0,30 g | 0,30 g | 0,30 g | 0,30 g |
| Pfefferextrakt | 0,10 g | 0,10 g | 0,10 g | 0,10g | 0,10 g |
| Trockenaroma | 1,00 g | 1,00 g | 1,00 g | 1,00 g | 1,00 g |
| Sprühgetrocknete Zusammensetzung gemäß Beispiel 1.2 | - | - | 0,70 g | 2,00 g | 0,70 g |

90 g der Soßenmischung werden mit 1000 ml heißem Wasser aufgegossen und mit dem Schneebesen kräftig verrührt.

### Anwendungsbeispiel 13: Tomatensuppe

A = Vergleichszubereitung
B = Vergleichszubereitung (Natriumglutamat-reduziert)
C = erfindungsgemäße Zubereitung (Natriumglutamat-reduziert)
D = erfindungsgemäße Zubereitung (Natriumglutamat-frei)
E = erfindungsgemäße Zubereitung (Natriumglutamat- und Salz-reduziert)

| **Bestandteil** | **A** | **B** | **C** | **D** | **E** |
|---|---|---|---|---|---|
| Wasser | 50,65 g | 50,80 g | 50,799 g | 51,035 g | 51,29 g |
| Pflanzenöl | 5,50 g | 5,50 g | 5,50 g | 5,50 g | 5,50 g |
| Tomatenpaste | 24,00 g | 24,00 g | 24,00 g | 24,00 g | 24,00 g |
| Sahne | 1,05 g | 1,05 g | 1,05 g | 1,05 g | 1,05 g |
| Zucker | 2,00 g | 2,00 g | 2,00 g | 2,00 g | 2,00 g |
| Kochsalz | 1,70 g | 1,70 g | 1,70 g | 1,70 g | 1,20 g |
| Natriumglutamat | 0,40 g | 0,25 g | 0,25 g | - | 0,25 g |
| Weizenmehl | 5,50 g | 5,50 g | 5,50 g | 5,50 g | 5,50 g |
| Stärke | 1,20 g | 1,20 g | 1,20 g | 1,20 g | 1,20 g |
| gewürfelte Tomaten | 8,00 g | 8,00 g | 8,00 g | 8,00 g | 8,00 g |
| Sprühgetrocknete Zusammensetzung gemäß Beispiel 1.1 | - | - | 0,001 g | 0,015 g | 0,010 g |

Die festen Bestandteile werden eingewogen, gemischt und dem Wasser hinzugefügt. Das Pflanzenöl wird zudosiert und die Tomatenpaste hinzugegeben. Die Mischung wird unter Rühren aufgekocht.

### Anwendungsbeispiel 14: Anwendung in einem Grünteegetränk

| **Inhaltsstoff** | **Einsatz in Gew.-%** | |
|---|---|---|
| | Ausführung A | Ausführung B |
| Grünteekonzentrat | 18.00 | 18.00 |
| 5% ige Lösung Rubemamin (1) Propylenglycol | 0.002 | - |
| 5% ige Lösung Rubescenamin (3) Propylenglycol | - | 0.002 |
| entmineralisiertes Wasser | 81.998 | 81.998 |

Das Grünteekonzentrat wird mit der jeweiligen 5 %igen Lösung des erfindungsgemäß zu verwendenden Zimtamids der Formel (I) in Propylenglycol vermischt. Anschließend wird mit entmineralisiertem Wasser aufgefüllt und erneut gründlich durchmischt. Dann wird das Produkt gefiltert, verbrauchsfertig verpackt und bei 118°C sterilisiert.

### Anwendungsbeispiel 15: Rindfleischwürzmischung für (Fertig)-Nudeln

| **Inhaltsstoff** | **Gew.-%** |
|---|---|
| Rindsfettaroma | 5 |
| Zuckercouleur | 3.00 |
| Zitronensäure (wasserfrei) | 0.40 |
| Schnittlauch (entwässert) | 2.00 |
| Maltodextrin (ex Tapoica) | 10.30 |
| Mononatriumglutamat | 15.00 |
| Zwiebelpulver | 5.00 |
| Ribotide | 0.80 |
| Natriumchlorid | 45.65 |
| Zucker | 2.80 |
| Süßmolkepulver | 6.50 |
| 10 % ige Lösung Rubemamin (1) in Propylenglycol | 0.05 |

Alle Inhaltsstoffe werden vermischt, bis sich eine homogene Mischung ergibt.

### Anwendungsbeispiel 17: (Fertig-)Nudeln

| **Teil** | **Inhaltsstoff** | **Gew.-%** |
|---|---|---|
| A | Weizenmehl | 62.00 |
| | Kartoffelstärke | 10.90 |
| B | Salz | 1.10 |
| | Guarkernmehl | 0.06 |
| | Natriumcarbonat | 0.07 |
| | Kaliumcarbonat | 0.25 |
| | Na₂H₂P₂O₇ | 0.07 |

| | | |
|---|---|---|
| | 10 % ige Lösung Rubescenamin (3) in Propylenglycol | 0.05 |
| C | Wasser | 25.45 |

Eine Suspension der Zutaten B in Wasser wird zu einer Mischung der Zutaten A gegeben und zu einem Teig geknetet. Nachdem der Teig für ca. 5 Minuten geruht hat, wird dieser mit Hilfe einer Nudelmaschine zu Platten verarbeitet, die in einem letzten Arbeitsschritt in eine übliche Form zurechtgeschnitten werden. Die Nudeln sind nach einer Kochzeit von 3 Minuten verzehrfertig und können z. B. mit 8 g der Rindfleischwürzmischung (Anwendungsbeispiel 16) angerichtet werden.

### Figurenbeschreibung:

**Fig. 1****:** Geschrn acklicher Vergleich von Rubemamin (1) mit Natriumglutamat.
   Der Geschmack eines 0,5 % amerikanischen Rindfleischextrakts als Base (i) wurde anhand einer Verkostung durch ein Panel ausgebildeter Testpersonen verglichen mit dem Geschmack einer solchen Base, der 10 ppm (iii) bzw. 50 ppm (iv) Rubemamin (1) zugesetzt wurden, und einer solchen Base, der 0,05 Gew.-% MSG zugesetzt wurden (ii).
   Die Testpersonen bewerteten die Stärke der angegebenen Geschmacksrichtungen jeweils durch Vergeben von Punktzahlen auf einer Skala von 0 (nicht wahrnehmbar) bis 9 (sehr starker entsprechender Geschmack). Dargestellt sind die Mittelwerte der jeweiligen Punktzahlen.
**Fig. 2****:** Geschmacklicher Vergleich von Rubescenamin (3) mit Natriumglutamat.
   Der Geschmack eines 0,5 % amerikanischen Rindfleischextrakts als Base (i) wurde anhand einer Verkostung durch ein Panel ausgebildeter Testpersonen verglichen mit dem Geschmack einer solchen Base, der 5 ppm (iii) bzw. 10 ppm (iv) Rubescenamin (3) zugesetzt wurden, und einer solchen Base, der 0,05 Gew.-% MSG zugesetzt wurden (ii).
   Die Testpersonen bewerteten die Stärke der angegebenen Geschmacksrichtungen jeweils durch Vergeben von Punktzahlen auf einer Skala von 0 (nicht wahrnehmbar) bis 9 (sehr starker entsprechender Geschmack). Dargestellt sind die Mittelwerte der jeweiligen Punktzahlen.
**Fig.3****:** Geschmacklicher Vergleich von Rubemamin (1) mit einem wenig Natriumglutamat-haltigen Rindfleischextrakt.
   Der Geschmack eines 0,5 % amerikanischen Rindfleischextrakts als Base (i) wurde anhand einer Verkostung durch ein Panel ausgebildeter Testpersonen verglichen mit dem Geschmack einer solchen Base, der 0,0025 % Natriumglutamat (iii) zugesetzt wurden, einer solchen Base, der 20 ppm Rubemamin (1) und 0.0025 % Natriumglutamat (iv) zugesetzt wurden, und einer solchen Base, der 0,05 Gew.-% MSG zugesetzt wurden (ii).
   Die Testpersonen bewerteten die Stärke der angegebenen Geschmacksrichtungen jeweils durch Vergeben von Punktzahlen auf einer Skala von 0 (nicht wahrnehmbar) bis 9 (sehr starker entsprechender Geschmack). Dargestellt sind die Mittelwerte der jeweiligen Punktzahlen.
**Fig.4****:** Geschmacklicher Vergleich von Rubescenamin (3) mit einem wenig Natriumglutamat-haltigen Rindfleischextrakt.
   Der Geschmack eines 0,5 % amerikanischen Rindfleischextrakts als Base (i) wurde anhand einer Verkostung durch ein Panel ausgebildeter Testpersonen verglichen mit dem Geschmack einer solchen Base, der 0,0025 % Natriumglutamat (iii) zugesetzt wurden, einer solchen Base, der 20 ppm Rubescenamin (3) und 0.0025 % Natriumglutamat (iv) zugesetzt wurden, und einer solchen Base, der 0,05 Gew.-% MSG zugesetzt wurde (ii).
   Die Testpersonen bewerteten die Stärke der angegebenen Geschmacksrichtungen jeweils durch Vergeben von Punktzahlen auf einer Skala von 0 (nicht wahrnehmbar) bis 9 (sehr starker entsprechender Geschmack). Dargestellt sind die Mittelwerte der jeweiligen Punktzahlen.
**Fig.5****:** Geschmacklicher Vergleich von Zanthomamin (9) mit einem wenig Natriumglutamat-haltigen Rindfleischextrakt.
   Der Geschmack eines 0,5 % amerikanischen Rindfleischextrakts als Base (i) wurde anhand einer Verkostung durch ein Panel ausgebildeter Testpersonen verglichen mit dem Geschmack erstens einer solchen Base, der 0,0025 % Natriumglutamat (iii) zugesetzt wurden, einer solchen Base, der 20 ppm Rubescenamin (3) und 0.0025 % Natriumglutamat (iv) zugesetzt wurden, und einer solchen Base, der 0,05 Gew.-% MSG zugesetzt wurden (ii).
   Die Testpersonen bewerteten die Stärke der angegebenen Geschmacksrichtungen jeweils durch Vergeben von Punktzahlen auf einer Skala von 0 (nicht wahrnehmbar) bis 9 (sehr starker entsprechender Geschmack). Dargestellt sind die Mittelwerte der jeweiligen Punktzahlen.

## Patentansprüche

1. Verwendung einer Verbindung der Formel (I) 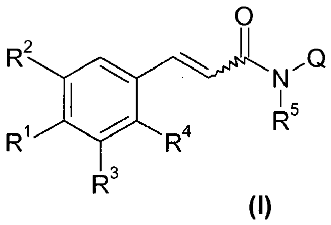 oder einer Mischung umfassend oder bestehend aus zwei oder mehr Verbindungen der Formel (I), wobei für die bzw. jede Verbindung der Formel (1) gilt:
- R¹ und R² stellen jeweils eine Methoxy-Gruppe dar
oder
R¹ und R² bilden zusammen eine Gruppe -O-CH₂-O-
oder
R¹ und R² stellen jeweils Wasserstoff dar,
- R³ und R⁴ stellen jeweils unabhängig voneinander Wasserstoff, eine Hydroxy-Gruppe oder eine Methoxy-Gruppe dar
- R⁵ stellt Wasserstoff oder eine Methyl-Gruppe dar,
- Q stellt eine Gruppe 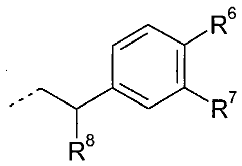
dar, wobei
R⁶ und R⁷ jeweils eine Methoxy-Gruppe darstellen oder zusammen eine Gruppe - O-CH₂-O- bilden
und R⁸ Wasserstoff, eine Hydroxy-Gruppe oder eine Methoxy-Gruppe darstellt, oder Q stellt eine Gruppe 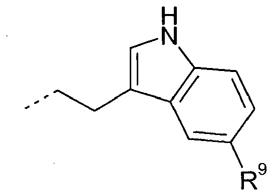 dar, wobei
R⁹ Wasserstoff, eine Hydroxy-Gruppe oder eine Methoxy-Gruppe darstellt,
als Geschmacksstoff bzw. Geschmacksstoffmischung zum Vermitteln, Modifizieren und/oder Verstärken einer, zwei oder sämtlicher der Geschmackseindrücke Umami, Kokumi und salzig.

2. Verwendung nach Anspruch 1, wobei für die bzw. eine, mehrere oder sämtliche Verbindungen der Formel (I) gilt:
- R¹ und R² stellen jeweils eine Methoxy-Gruppe dar
oder
R¹ und R² bilden zusammen eine Gruppe -O-CH₂-O-
oder
R¹ und R² stellen jeweils Wasserstoff dar,
- R³ und R⁴ stellen jeweils unabhängig voneinander Wasserstoff dar
- R⁵ stellt Wasserstoff oder eine Methyl-Gruppe dar,
- Q stellt eine Gruppe 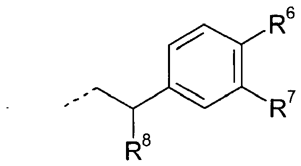 dar, wobei
R⁶ und R⁷ jeweils eine Methoxy-Gruppe darstellen oder zusammen eine Gruppe-O-CH₂-O- bilden
und R⁸ Wasserstoff darstellt.

3. Verwendung nach einem der Ansprüche 1 oder 2, wobei die Verbindung der Formel (I) bzw. eine, mehrere oder sämtliche der Verbindungen der Formel (I) ausgewählt ist bzw. sind aus der Gruppe bestehend aus
Verbindung (1) der Formel 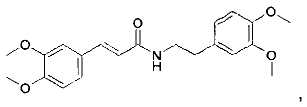 Verbindung (2) der Formel 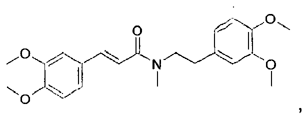 Verbindung (3) der Formel 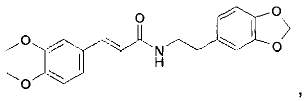 Verbindung (4) der Formel 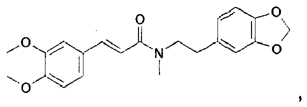 Verbindung (5) der Formel 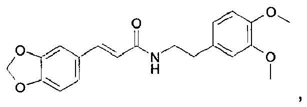 Verbindung (6) der Formel 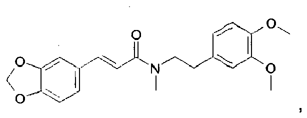 Verbindung (7) der Formel 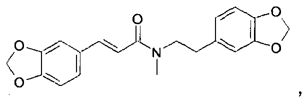 Verbindung (8) der Formel 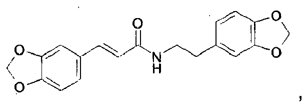 Verbindung (9) der Formel 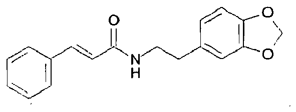 Verbindung (10) der Formel

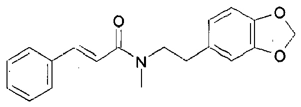

4. Verwendung nach einem der vorangehenden Ansprüche, wobei die Verbindung der Formel (I) bzw. beide Verbindungen der Formel (I) bzw. eine oder zwei der Verbindungen der Formel (I) ausgewählt ist bzw. sind aus der Gruppe bestehend aus
Verbindung (1) der Formel 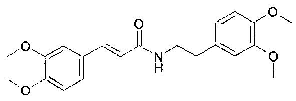 Verbindung (3) der Formel

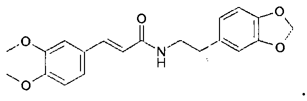

5. Geschmacksstoffmischung umfassend oder bestehend aus
(i) einer Verbindung der Formel (I) oder einer Mischung umfassend oder bestehend aus zwei oder mehr Verbindungen der Formel (I), wie jeweils in einem der Ansprüche 1 bis 4 definiert, vorzugsweise wie in Anspruch 4 definiert,
sowie
(ii) einem, zwei, drei oder mehreren weiteren Geschmacksstoffen zum Vermitteln, Modifizieren und/oder Verstärken einer, zwei oder sämtlicher der Geschmackseindrücke Umami, Kokumi und salzig, wobei der bzw. die weiteren Geschmacksstoffe keine Verbindungen der Formel (I) sind.

6. Geschmacksstoffmischung nach Anspruch 5, wobei der bzw. einer, zwei, drei, mehrere oder sämtliche der weiteren Geschmacksstoffe (ii) ausgewählt sind aus der Gruppe bestehend aus Mononatriumglutamat, freie Glutaminsäure, Nucleotide oder deren pharmazeutisch akzeptable Salze, Strombine, Theogalline, Pyridin-Betain-Verbindungen, Glutaminsäureglycoside, Äpfelsäureglycoside, Glutathion-Derivate, Lactisole und Alkylpyridine, insbesondere 2-Hexyl-, 2-Heptyl und 2-Octylpyridin, (2E,6Z)-N-cyclopropylnona-2,6-dienamid, (2E,6Z)-N-ethylnona-2,6-dienamid, N-[(2E)-3,7-dimethylocta-2,6-dienyl]cyclopropancarboxamid, N'-[(2-methoxy-4-methyl-phenyl)methyl]-N-[2-(5-methyl-2-pyridyl)ethyl]oxamid, N'-[(2,4-dimethoxyphenyl)methyl]-N-[2-(2-pyridyl)ethyl]oxamid, N'-[(2-methoxy-4-methyl-phenyl)methyl]-N-[2-(2-pyridyl)ethyl]oxamid, N-(1-propylbutyl)-1,3-benzodioxole-5-carboxamid, 1-(2-hydroxy-4-isobutoxy-phenyl)-3-(2-pyridyl)propan-1-on und 1-(2-hydroxy-4-methoxy-phenyl)-3-(2-pyridyl)propan-1-on.

7. Geschmacksstoffmischung nach einem der Ansprüche 5 oder 6, wobei die Gesamtmenge an Verbindungen der Formel (I) bezogen auf das Gesamtgewicht der Geschmacksstoffmischung im Bereich von 0,0001 bis 95 Gew.-% liegt.

8. Pflanzlicher Extrakt umfassend oder bestehend aus
- einer Verbindung der Formel (I) oder einer Mischung umfassend oder bestehend aus zwei oder mehr Verbindungen der Formel (I), wie jeweils in einem der Ansprüche 1 bis 4 definiert, vorzugsweise wie in Anspruch 4 definiert,
sowie
- einem oder mehreren weiteren Bestandteilen,
wobei die Gesamtmenge an Verbindungen der Formel (I) bezogen auf das Gesamtgewicht des pflanzlichen Extrakts im Bereich von 1.000 bis 500.000 ppm, vorzugsweise im Bereich von 10.000 bis 100.000 ppm liegt.

9. Verwendung eines pflanzlichen Extrakts nach Anspruch 8 zum Vermitteln, Modifizieren und/oder Verstärken einer, zwei oder sämtlicher der Geschmackseindrücke Umami, Kokumi und salzig.

10. Der Ernährung oder dem Genuss dienende gebrauchs- oder verzehrfertige Zubereitung umfassend oder bestehend aus
(a1) einer Verbindung der Formel (I) oder einer Mischung umfassend oder bestehend aus zwei oder mehr Verbindungen der Formel (I), wie jeweils in einem der Ansprüche 1 bis 4 definiert, vorzugsweise wie in Anspruch 4 definiert,
sowie
(b) einem oder mehreren weiteren zum Verzehr geeigneten Bestandteilen, wobei der bzw. die weiteren Bestandteile keine Verbindungen der Formel (I) sind,
wobei die Gesamtmenge an Verbindungen der Formel (I) bezogen auf das Gesamtgewicht der gebrauchs- oder verzehrfertigen Zubereitung im Bereich von 5 ppm bis 500 ppm, bevorzugt im Bereich von 5 ppm bis 200 ppm, insbesondere bevorzugt im Bereich von 5 ppm bis 100 ppm liegt.

11. Der Ernährung oder dem Genuss dienende gebrauchs- oder verzehrfertige Zubereitung umfassend oder bestehend aus
(a2) einer Geschmackstoffmischung nach einem der Ansprüche 5 bis 7 oder
einem pflanzlichen Extrakt nach Anspruch 8
sowie
(b) einem oder mehreren weiteren zum Verzehr geeigneten Bestandteilen, wobei der bzw. die weiteren Bestandteile keine Verbindungen der Formel (I) sind.

12. Zubereitung nach Anspruch 11, wobei die Gesamtmenge an Verbindungen der Formel (I) bezogen auf das Gesamtgewicht der Zubereitung im Bereich von 5 ppm bis 500 ppm, bevorzugt im Bereich von 5 ppm bis 200 ppm, insbesondere bevorzugt im Bereich von 5 ppm bis 100 ppm liegt.

13. Halbfertigware zur Herstellung einer der Ernährung oder dem Genuss dienenden Zubereitungen, vorzugsweise einer Zubereitung nach einem der Ansprüche 10 bis 12, umfassend oder bestehend aus
(a) einer Verbindung der Formel (I) oder einer Mischung umfassend oder bestehend aus zwei oder mehr Verbindungen der Formel (I), wie jeweils in einem der Ansprüche 1 bis 4 definiert, vorzugsweise wie in Anspruch 4 definiert,
oder
einer Geschmackstoffmischung nach einem der Ansprüche 5 bis 7
oder
einem pflanzlichen Extrakt nach Anspruch 8
sowie
(b) einem oder mehreren weiteren zum Verzehr geeigneten Bestandteilen, wobei der bzw. die weiteren Bestandteile keine Verbindungen der Formel (I) sind,
wobei die Gesamtmenge an Verbindungen der Formel (I) bezogen auf das Gesamtgewicht der Halbfertigware im Bereich von 10 ppm bis 500.000 ppm, bevorzugt im Bereich von 25 ppm bis 100.000 ppm, insbesondere im Bereich von 50 ppm bis 15.000 ppm, liegt.

14. Zubereitung oder Halbfertigware nach einem der Ansprüche 10 bis 13, wobei die Zubereitung bzw. Halbfertigware eine Natriumglutamat-freie Zubereitung bzw. Halbfertigware oder eine Natriumglutamat-reduzierte Zubereitung bzw. Halbfertigware ist.

15. Natriumglutamat-reduzierte Zubereitung nach Anspruch 14, wobei das Gewichtsverhältnis der Gesamtmenge an Verbindungen der Formel (I) zur Gesamtmenge an Natriumglutamat, bezogen auf die Trockenmasse der Zubereitung, im Bereich von 1 : 1 bis 1 : 200 liegt.

## Claims

1. Use of a compound of formula (I) 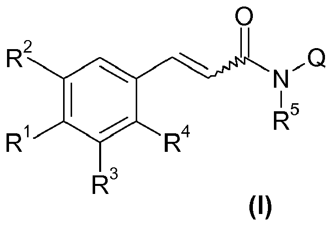 or a mixture comprising or consisting of two or more compounds of formula (I), wherein for the or for each compound of formula (I), respectively, the following applies:
- R¹ and R² each represent a methoxy group
or
R¹ and R² together form a group -O-CH₂-O-
or
R¹ and R² each represent hydrogen,
- R³ and R⁴ each, independent of each other, represent hydrogen, a hydroxy group or a methoxy group
- R⁵ represents hydrogen or a methyl group,
- Q represents a group 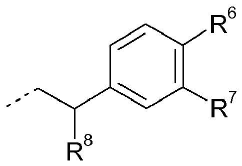
wherein
R⁶ and R⁷ each represent a methoxy group or together form a group -O-CH₂-O-
and R⁸ represents hydrogen, a hydroxy group or a methoxy group,
or Q represents a group 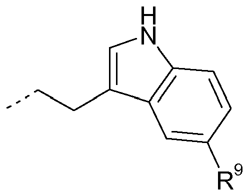 wherein
R⁹ represents hydrogen, a hydroxy group or a methoxy group,
as a flavor or a mixture of flavors, respectively, to convey, modify and/or enhance one, two or all of the taste impressions umami, kokumi and salty.

2. Use according to claim 1, wherein for the or for one or all of the compounds of formula (I), respectively, the following applies:
- R¹ and R² each represent a methoxy group
or
R¹ and R² together form a group -O-CH₂-O-
or
R¹ and R² each represent hydrogen,
- R³ and R⁴ each, independent of each other, represent hydrogen,
- R⁵ represents hydrogen or a methyl group,
- Q represents a group 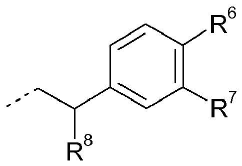 wherein
R⁶ and R⁷ each represent a methoxy group or together form a group -O-CH₂-O-
and R⁸ represents hydrogen.

3. Use according to one of the claims 1 or 2, wherein the compound of formula (I) or one, several or all of the compounds of formula (I), respectively, is/are selected from the group consisting of
compound (1) of formula 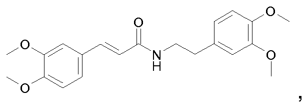 compound (2) of formula 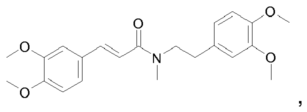 compound (3) of formula 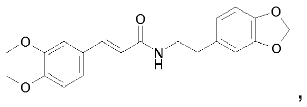 compound (4) of formula 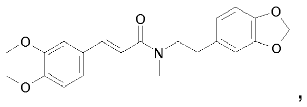 compound (5) of formula 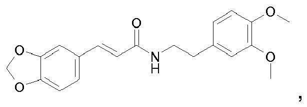 compound (6) of formula 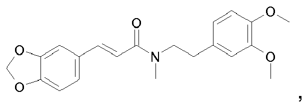 compound (7) of formula 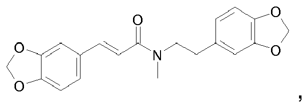 compound (8) of formula 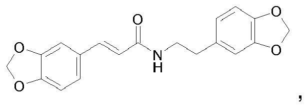 compound (9) of formula 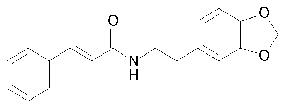 and
compound (10) of formula

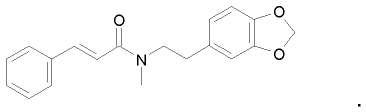

4. Use according to one of the previous claims, wherein the compound of formula (I) or both compounds of formula (I) or one or two of the compounds of formula (I) is/are selected from the group consisting of
compound (1) of formula 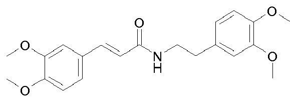 and
compound (3) of formula

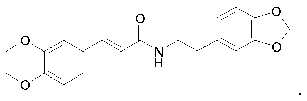

5. Mixture of flavors comprising or consisting of
(i) a compound of formula (I) or a mixture comprising or consisting of two or more compounds of formula (I), as defined in any one of the claims 1 to 4, preferably as defined in claim 4,
as well as
(ii) one, two, three or more further flavors to convey, modify and/or enhance one, two or all of the taste impressions umami, kokumi and salty, wherein the one or the further flavor(s) is/are not compounds of formula (I).

6. Mixture of flavors according to claim 5, wherein the or one, two, three, several or all of the further flavors (ii) are selected from the group consisting of monosodium glutamate, free glutamic acid, nucleotides or pharmaceutically acceptable salts thereof, strombines, theogallines, pyridine betaine compounds, glutamic acid glycosides, malic acid glycosides, glu-tathione derivates, lactisoles and alkyl pyridines, in particular 2-hexyl, 2-heptyl and 2-octyl pyridine, (2E,6Z)-N-cyclopropylnona-2,6-dienamide, (2E,6Z)-N-ethylnona-2,6-dienamide, N-[(2E)-3,7-dimethylocta-2,6-dienyl]cyclopropan carboxamide, N'-[(2-methoxy-4-methyl-phenyl)methyl]-N-[2-(5-methyl-2-pyridyl)ethyl]oxamide, N'-[(2,4-dimethoxyphenyl)methyl]-N-[2-(2-pyridyl)ethyl]oxamide, N'-[(2-methoxy-4-methyl-phenyl)methyl]-N-[2-(2-pyridyl)ethyl]oxamide, N-(1 -propylbutyl)-1,3-benzodioxole-5-carboxamide, 1-(2-hydroxy-4-isobutoxy-phenyl)-3-(2-pyridyl)propan-1-one und 1-(2-hydroxy-4-methoxy-phenyl)-3-(2-pyridyl)propan-1-one.

7. Mixture of flavors according to one of claims 5 or 6, wherein the total amount of compounds of formula (I) with respect to the total weight of the mixture of flavors is in the range from 0.0001 to 95 wt.-%.

8. Plant extract comprising or consisting of
- a compound of formula (I) or a mixture comprising or consisting of two or more compounds of formula (I), as defined in any one of the claims 1 to 4, preferably as defined in claim 4,
as well as
- one or more further components,
wherein the total amount of compounds of formula (I) with respect to the total weight of the plant extract is in the range from 1.000 to 500.000 ppm, preferably in the range from 10.000 to 100.000 ppm.

9. Use of a plant extract according to claim 8 to convey, modify and/or enhance one, two or all of the taste impressions umami, kokumi and salty.

10. Preparation for nutrition or pleasure ready for use or consumption comprising or consisting of
(a1) a compound of formula (I) or a mixture comprising or consisting of two or more compounds of formula (I), as defined in any one of the claims 1 to 4, preferably as defined in claim 4,
as well as
(b) one or more further components suitable for consumption, wherein the one or the further component(s) is/are not compounds of formula (I),
wherein the total amount of compounds of formula (I) with respect to the total weight of the preparation ready for use or consumption is in the range from 5 ppm to 500 ppm, preferably in the range from 5 ppm to 200 ppm, particularly preferably in the range from 5 ppm to 100 ppm.

11. Preparation for nutrition or pleasure ready for use or consumption comprising or consisting of
(a2) a mixture of flavors according to one of claims 5 to 7
or
a plant extract according to claim 8
as well as
(b) one or more further components suitable for consumption, wherein the one or the further component(s) is/are not compounds of formula (I).

12. Preparation according to claim 11, wherein the total amount of compounds of formula (I) with respect to the total weight of the preparation is in the range from 5 ppm to 500 ppm, preferably in the range from 5 ppm to 200 ppm, particularly preferably in the range from 5 ppm to 100 ppm.

13. Semi-finished product for the production of a preparation for nutrition or pleasure, preferably a preparation according to one of claims 10 to 12, comprising or consisting of
(a) a compound of formula (I) or a mixture comprising or consisting of two or more compounds of formula (I), as defined in any one of the claims 1 to 4, preferably as defined in claim 4,
or
a mixture of flavors according to one of the claims 5 to 7
or
a plant extract according to claim 8
as well as
(b) one or more further components suitable for consumption, wherein the one or the further component(s) is/are not compounds of formula (I),
wherein the total amount of compounds of formula (I) with respect to the total weight of the semi-finished product is in the range from 10 ppm to 500.000 ppm, preferably in the range from 25 ppm to 100.000 ppm, particularly in the range from 50 ppm to 15.000 ppm.

14. Preparation or semi-finished product according to one of the claims 10 to 13, wherein the preparation or the semi-finished product is a sodium glutamate free preparation or semi-finished product, respectively, or a sodium glutamate reduced preparation or semi-finished product, respectively.

15. Sodium glutamate reduced preparation according to claim 14, wherein the weight ratio of the total amount of compounds of formula (I) to the total amount of sodium glutamate, with respect to the dry mass of the preparation, is in the range from 1 : 1 to 1 : 200.

## Revendications

1. Utilisation d'un composé de la formule (I) 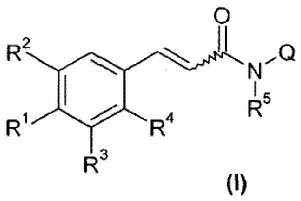 ou d'un mélange comprenant ou se composant de deux composés ou plus de la formule (I), pour ledit ou bien chaque composé de la formule (I) valant :
- R¹ et R² représentent respectivement un groupe méthoxy
ou
R¹ et R² forment ensemble un groupe -O-CH₂-O-
ou
R¹ et R² représentent respectivement de l'hydrogène,
- R³ et R⁴ représentent respectivement, indépendamment l'un de l'autre, de l'hydrogène, un groupe hydroxy ou un groupe méthoxy,
- R⁵ représente de l'hydrogène ou un groupe méthyle,
- Q représente un groupe 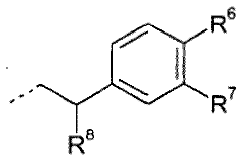 dans lequel
R⁶ et R⁷ représentent respectivement un groupe méthoxy ou forment ensemble un groupe -O-CH₂-O-
et R⁸ représente de l'hydrogène, un groupe hydroxy ou un groupe méthoxy ou Q représente un groupe 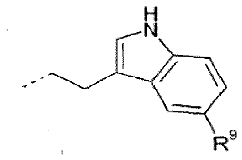 dans lequel
R⁹ représente de l'hydrogène, un groupe hydroxy ou un groupe méthoxy, comme agent de sapidité ou bien mélange d'agents de sapidité, pour communiquer, modifier et/ou renforcer une, deux ou l'ensemble des impressions de goût umami, kokumi et salé.

2. Utilisation selon la revendication 1, pour ledit ou bien un, plusieurs ou tous les comosés de la formule (I) valant :
- R¹ et R² représentent respectivement un groupe méthoxy
ou
R¹ et R² forment ensemble un groupe -O-CH₂-O-
ou
R¹ et R² représentent respectivement de l'hydrogène,
- R³ et R⁴ représentent respectivement, indépendamment l'un de l'autre, de l'hydrogène,
- R⁵ représente de l'hydrogène ou un groupe méthyle,
- Q représente un groupe 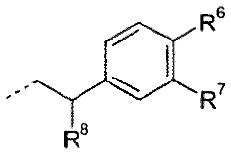 dans lequel R⁶ et R⁷ représentent respectivement un groupe méthoxy ou forment ensemble un groupe -O-CH₂-O-
et R⁸ représente de l'hydrogène.

3. Utilisation selon l'une quelconque des revendications 1 ou 2, dans laquelle le composé de la formule (I) ou bien un, plusieurs ou l'ensemble des composés de la formule (I) est ou bien sont choisi(s) dans le groupe se composant de
composé (1) de la formule 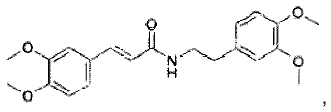 composé (2) de la formule 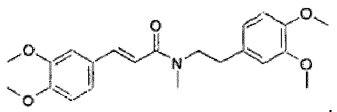 composé (3) de la formule 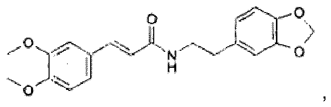 composé (4) de la formule 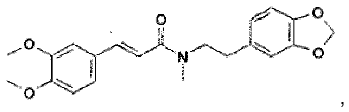 composé (5) de la formule 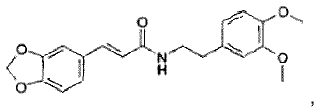 composé (6) de la formule 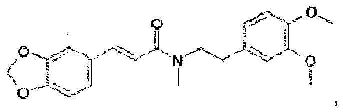 composé (7) de la formule 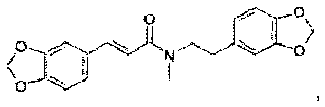 composé (8) de la formule 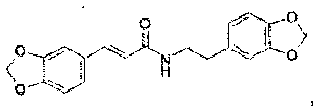 composé (9) de la formule 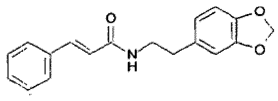 et
composé (10) de la formule

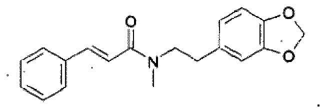

4. Utilisation selon l'une quelconque des revendications précédentes, dans laquelle le composé de la formule (I) ou bien les deux composés de la formule (I) ou bien un ou deux des composés de la formule (I) est ou bien sont choisi(s) dans le groupe se composant de
composé (1) de la formule 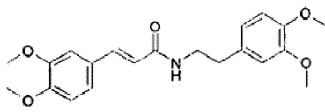 et
composé (3) de la formule

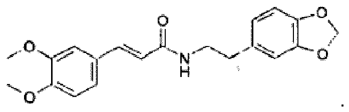

5. Mélange d'agents de sapidité comprenant ou se composant de
(i) un composé de la formule (I) ou un mélange comprenant ou se composant de deux composés ou plus de la formule (I), tel que défini respectivement dans l'une quelconque des revendications 1 à 4, de préférence tel que défini dans la revendication 4,
ainsi que
(ii) un, deux, trois ou plusieurs autres agents de sapidité pour communiquer, modifier et/ou renforcer une, deux ou l'ensemble des impressions de goût umami, kokumi et salé, ledit ou bien lesdits autres agents de sapidité n'étant pas des composés de la formule (I).

6. Mélange d'agents de sapidité selon la revendication 5, dans lequel ledit ou bien un, deux, trois, plusieurs ou l'ensemble des autres agents de sapidité (ii) sont choisis dans le groupe se composant du monoglutamate de sodium, de l'acide glutamique libre, des nucléotides ou de leurs sels pharmaceutiquement acceptables, des strombines, des théogallines, des composés de pyridine-bétaïne, des glycosides de l'acide glutamique, des glycosides de l'acide malique, des dérivés de glutathion, des lactisoles et alkylpyridines, en particulier la 2-hexyl-, 2-heptyl- et 2-octyl-pyridine, (2E,6Z)-N-cyclopropylnona-2,6-diénamide, (2E,6Z)-N-éthylnona-2,6-diénamide, N-[(2E)-3,7-diméthylocta-2,6-diényl]cyclopropancarboxamide, N'-[(2-méthoxy-4-méthyl-phényl)méthyl]-N-[2-(5-méthyl-2-pyridyl)éthyl]oxamide, N'-[(2,4-diméthoxyphényl)méthyl]-N-[2-(2-pyridyl)éthyl]oxamide, N'-[(2-méthoxy-4-méthyl-phényl)méthyl]-N-[2-(2-pyridyl)éthyl]oxamide, N-(1-propylbutyl)-1,3-benzodioxole-5-carboxamide, 1-(2-hydroxy-4-isobutoxy-phényl)-3-(2-pyridyl)propan-1-one et 1-(2-hydroxy-4-méthoxy-phényl)-3-(2-pyridyl)propan-1-one.

7. Mélange d'agents de sapidité selon l'une quelconque des revendications 5 ou 6, dans lequel la quantité totale de composés de la formule (I) par rapport au poids total du mélange d'agents de sapidité est comprise entre 0,0001 et 95 % en poids.

8. Extrait végétal comprenant ou se composant de
- un composé de la formule (I) ou un mélange comprenant ou se composant de deux composés ou plus de la formule (I), tel que défini respectivement dans l'une quelconque des revendications 1 à 4, de préférence tel que défini dans la revendication 4,
ainsi que
(ii) un ou plusieurs autres composants,
dans lequel la quantité totale de composés de la formule (I) par rapport au poids total dudit extrait végétal est comprise entre 1.000 et 500.000 ppm, de préférence entre 10.000 et 100.000 ppm.

9. Utilisation d'un extrait végétal selon la revendication 8, pour communiquer, modifier et/ou renforcer une, deux ou l'ensemble des impressions de goût umami, kokumi et salé.

10. Préparation prête à être utilisée ou consommée qui sert à l'alimentation ou au plaisir, comprenant ou se composant de
(a1) un composé de la formule (I) ou un mélange comprenant ou se composant de deux composés ou plus de la formule (I), tel que défini respectivement dans l'une quelconque des revendications 1 à 4, de préférence tel que défini dans la revendication 4,
ainsi que
(b) un ou plusieurs autres composants aptes à être consommés, ledit ou bien lesdits autres composants n'étant pas des composés de la formule (I),
dans laquelle la quantité totale de composés de la formule (I) par rapport au poids total de la préparation prête à être utilisée ou consommée est comprise entre 5 ppm et 500 ppm, de préférence entre 5 ppm et 200 ppm, en particulier de préférence entre 5 ppm et 100 ppm.

11. Préparation prête à être utilisée ou consommée qui sert à l'alimentation ou au plaisir, comprenant ou se composant de
(a2) un mélange d'agents de sapidité selon l'une quelconque des revendications 5 à 7,
ou
un extrait végétal selon la revendication 8
ainsi que
(b) un ou plusieurs autres composants aptes à être consommés, ledit ou bien lesdits autres composants n'étant pas des composés de la formule (I).

12. Préparation selon la revendication 11, dans laquelle la quantité totale de composés de la formule (I) par rapport au poids total de la préparation est comprise entre 5 ppm et 500 ppm, de préférence entre 5 ppm et 200 ppm, en particulier de préférence entre 5 ppm et 100 ppm.

13. Produit semi-fini pour la production d'une préparation servant à l'alimentation ou au plaisir, de préférence d'une préparation selon l'une quelconque des revendications 10 à 12, comprenant ou se composant de
(a) un composé de la formule (I) ou un mélange comprenant ou se composant de deux composés ou plus de la formule (I), tel que défini respectivement dans l'une quelconque des revendications 1 à 4, de préférence tel que défini dans la revendication 4,
ou
un mélange d'agents de sapidité selon l'une quelconque des revendications 5 à 7 ou
un extrait végétal selon la revendication 8
ainsi que
(b) un ou plusieurs autres composants aptes à être consommés, ledit ou bien lesdits autres composants n'étant pas des composés de la formule (I),
dans lequel la quantité totale de composés de la formule (I) par rapport au poids total du produit semi-fini est comprise entre 10 ppm et 500.000 ppm, de préférence entre 25 ppm et 100.000 ppm, en particulier entre 50 ppm et 15.000 ppm.

14. Préparation ou produit semi-fini selon l'une quelconque des revendications 10 à 13, ladite préparation ou bien ledit produit semi-fini étant une préparation ou bien un produit semi-fini exempt(e) de glutamate de sodium ou une préparation ou bien un produit semi-fini à teneur réduite en glutamate de sodium.

15. Préparation à teneur réduite en glutamate de sodium, selon la revendication 14, dans laquelle le rapport en poids de la quantité totale de composés de la formule (I) à la quantité totale de glutamate de sodium, par rapport à la matière sèche de la préparation, est compris entre 1 : 1 et 1 : 200.
